# EUROPEAN PATENT SPECIFICATION

(11) **EP 1 611 250 B1**
(45) Date of publication and mention of the grant of the patent: **19.12.2012**
(21) Application number: 04720803.8
(22) Date of filing: 15.03.2004
(51) Int. Cl.: G01N 33/574

(54) **DETECTION OF URINARY MESOTHELIN-/MEGAKARYOCYTE POTENTIATING FACTOR-RELATED PEPTIDES FOR ASSESSMENT OF OVARIAN CANCER**
NACHWEIS VON HARNMESOTHELIN-/MEGAKARYOZYTPOTENZIERUNGSFAKTOR-PEPTIDEN ZUR BEURTEILUNG EINES OVARIALKARZINOMS
DETECTION DE PEPTIDES LIES AU FACTEUR DE POTENTIALISATION DE MEGACARYOCYTE/MESOTHELINE URINAIRE DANS LA DETECTION DU CANCER DE L'OVAIRE

(30) Priority: 13.03.2003 US 388930
(43) Date of publication of application: 04.01.2006
(60) Divisional application: 10012291.0
(73) Proprietor: Fujirebio America, Inc., Wilmington, DE 19808 (US)
(72) Inventor: O'SHANNESSY, Daniel, J., Limerick, PA 19468 (US); SARDESAI, Niranjan, Y., North Wales, PA 19454 (US); BONES, Jennifer, West Chester, PA 19382 (US)
(74) Representative: Grünecker, Kinkeldey, Stockmair & Schwanhäusser
(86) International application number: PCT/US2004/007765
(87) International publication number: WO 2004/083449

(56) References cited:
- EP-B1- 1 169 645
- WO-A2-02/071928
- US-A- 6 083 502
- US-A1- 2003 087 250
- SCHOLLER N ET AL: "Soluble member(s) of the mesothelin /megakaryocyte potentiating factor family are detectable in sera from patients with ovarian carcinoma" PROCEEDINGS OF THE NATIONAL ACADEMY OF SCIENCES OF USA, NATIONAL ACADEMY OF SCIENCE, WASHINGTON, DC, US, vol. 96, no. 20, 28 September 1999 (1999-09-28), pages 11531-11536, XP002153470 ISSN: 0027-8424
- TAY S K ET AL: "Correlation of serum, urinary and salivary CA 125 levels in patients with adnexal masses." ANNALS OF THE ACADEMY OF MEDICINE, SINGAPORE. MAY 1994, vol. 23, no. 3, May 1994 (1994-05), pages 311-314, XP009076323 ISSN: 0304-4602
- ARGANI P ET AL: "Mesothelin is overexpressed in the vast majority of ductal adenocarcinomas of the pancreas: identification of a new pancreatic cancer marker by serial analysis of gene expression (SAGE)." CLINICAL CANCER RESEARCH : AN OFFICIAL JOURNAL OF THE AMERICAN ASSOCIATION FOR CANCER RESEARCH. DEC 2001, vol. 7, no. 12, December 2001 (2001-12), pages 3862-3868, XP009076502 ISSN: 1078-0432
- PATTARI SANJIB KUMAR ET AL: "Urine: beyond cytology for detection of malignancy." DIAGNOSTIC CYTOPATHOLOGY. SEP 2002, vol. 27, no. 3, September 2002 (2002-09), pages 139-142, XP009076504 ISSN: 8755-1039
- STENMAN U-H ET AL: "MARKERS SUPPLEMENTING CA 125 IN OVARIAN CANCER" ANNALS OF MEDICINE, FINNISH MEDICAL SOCIETY DUODECIM, HELSINKI, FI, vol. 27, no. 1, 1995, pages 115-120, XP009007535 ISSN: 0785-3890
- KOS J ET AL: "CYSTEINE PROTEINASES AND THEIR ENDOGENOUS INHIBITORS: TARGET PROTEINS FOR PROGNOSIS, DIAGNOSIS AND THERAPY IN CANCER (REVIEW)" ONCOLOGY REPORTS, NATIONAL HELLENIC RESEARCH FOUNDATION, ATHENS, GR, vol. 5, no. 6, November 1998 (1998-11), pages 1349-1361, XP001030675 ISSN: 1021-335X
- CRUMP C. ET AL: 'Ovarian Cancer Tumor Marker Behavior in Asymptomatic Healthy Women: Implications for Screening' CANCER EPIDEMIOLOGY, BIOMARKERS AND PREVENTION vol. 9, October 2000, pages 1107 - 1111, XP002988084
- BRINKMANN U. ET AL: 'Cloning and expression of the recombinant FAb fragment of monoclonal antibody K1 that reacts with mesothelin present on mesotheliomas and ovarian cancers' INT J CANCER vol. 71, no. 4, 16 May 1997, pages 638 - 644, XP002102002

## Description

### BACKGROUND OF THE INVENTION

The invention relates generally to diagnosing ovarian cancer in women.

Ovarian cancer is one of the foremost causes of cancer deaths among women in the U.S. One reason for the relatively high morality rate among ovarian cancer patients is that the disease is often not diagnosed until it reaches an advanced phase.

An ovarian tumor can be classified by the cell type from which the tumor arose, by the morphology of cells of the tumor, and by the stage to which the tumor has progressed.

Ovarian tumors can arise from any of three ovarian cell types - epithelial, germ cells, and stromal cells. Epithelial ovarian tumors (EOTs) are the most common type of ovarian tumor, accounting for 85 percent or more of all ovarian tumors. EOTs arise from the epithelial cells that cover the outer surface of the ovary. Some EOTs are benign and cause relatively little medical harm. However, malignant EOTs can grow uncontrollably, invade nearby tissues, or metastasize to distant body locations. Germ cell ovarian tumors arise from ova-producing cells and can also be either benign or malignant. Stromal ovarian tumors arise from ovary connective tissue cells and are relatively rare.

EOTs are further characterized by the type of cells that make up the EOT. Recognized cell types include serous, mucinous, endometrioid, and clear cell types. Some EOTs are not clearly differentiated among these cell types, and these non-differentiated EOTs tend to grow and spread more quickly than more clearly differentiated types of EOTs.

EOTs are also classified by grade and stage. The grade of a tumor describes its macroscopic appearance. Grade 1 tumors tend to closely resemble normal ovarian tissue, and also tend to have a better prognosis. Grade 3 tumors tend to have an abnormal appearance, and usually have a worse prognosis. Grade 2 tumors are intermediate between Grades 1 and 3. The stage of a tumor describes the degree or extent to which the tumor appears to have spread from its origin. Stage I ovarian tumors are limited to one or both ovaries. Gradations within Stage I describe tumors limited to the interior of one (Stage IA) or both ovaries (Stage IB) and tumors (Stage IC) which appear on the surface of one or both ovaries, in one or more ovaries having a ruptured capsule, or in association with tumor cells present in ascites or detected in a peritoneal wash. Stage II ovarian tumors have extended within the pelvis beyond the ovaries. Gradations within Stage II describe tumors that have extended to one or both of the uterus and the fallopian tubes (Stage IIA), to other pelvic organs (Stage IIB), or which are associated with tumor cells present in ascites or detected in a peritoneal wash. Stage III ovarian tumors have spread beyond the pelvis, to the lymph nodes, or both. Stage IV ovarian tumors have spread to organs beyond the peritoneal cavity.

Early diagnosis of an ovarian tumor is important for effective treatment of the tumor and for extending patient survival. Early diagnosis of ovarian cancer is complicated by the fact that there are few macroscopic symptoms until the disease has progressed to an advanced phase. Existing diagnostic tests for ovarian tumors are not universally successful for detecting ovarian cancer at a sufficiently early phase that efficacious treatment can be administered. The reasons that existing tests do not detect ovarian cancer sufficiently early are not completely known. For example, some tests may rely on markers that are released from tumor cells at levels that are too low to be reliably detected until the tumor mass has grown relatively large.

Markers that have been used by others to diagnose ovarian cancer include CA 125 (Halila et al., 1987, Br. J. Cancer 56(2):153-156), carcinoembryonic antigen (CEA; Juweid et al., 1997, Gynecol. Oncol. 67(3):259-271), vascular endothelial growth factor (VEGF; Candido Dos Reis et al., 2002, Gynecol. Obstet. Invest. 54(3):132-136), urinary gonadotropin peptide (UGP; Schutter et al., 1999, Anticancer Res. 19(6C):5551-5557), neopterin (Szarka et al., 1988, Acta Chir. Hung. 29(4):359-364), CD44 splice variants (Uhl-Steidl et al., 1995, Oncology 52(5):400-406), urine cysteine proteinase (UCP; Gao et al., 1996, Hua Xi Yi Ke Da Xue Xue Bao 27(3):291-294), anti-malignin antibody (Bogoch et al., 1991, Lancet 337:927), and tumor-associated trypsin inhibitor (TATI; Medl et al., 1995, Br. J. Cancer 71(5):1051-1054; Halila et al., 1988, Br. J. Cancer 57(3):304-307). Kudoh et al., (1999, Gynecol. Obstet. Invest. 47(1);52-57) compared the usefulness of several other serum markers for EOTs, including lactate dehydrogenase (LDH), alpha-hydroxybutyrate dehydrogenase (aHBDH), CA19-9, tissue polypeptide antigen (TPA), and sialyl TN (STN). Every markers listed in this paragraph is not present in abnormal amounts in every ovarian cancer patient.

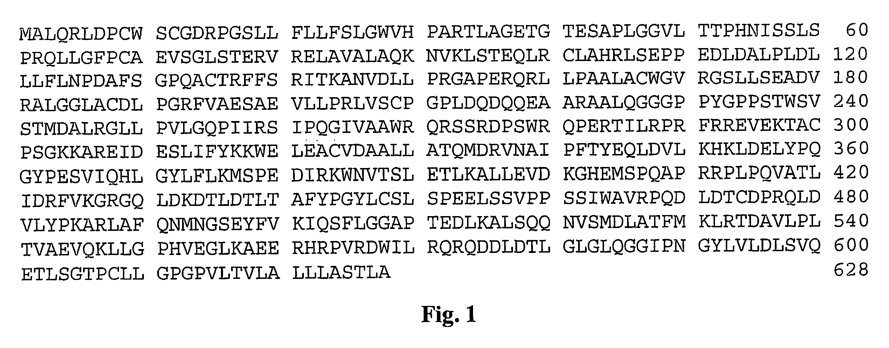

Mesothelin is a protein which is expressed on the surface of at least mesothelial cells, mesotheliomas, some squamous cell carcinomas, and ovarian cancers (Chang et al., 1996, Proc. Natl. Acad. Sci. USA 93:136-140). Mesothelin has been described in the literature (e.g., U.S. Patent No. 6,083,502). Antibodies that bind with mesothelin have also been described. For example, a monoclonal antibody designated K1 is described in U.S. Patent No. 5,320,956, and is secreted by the hybridoma that was deposited by others with the American Type Culture Collection (ATCC; 10801 University Boulevard; Manassas, VA 20110-2209; USA) as accession number HB 10570. Antibody K1 was found to bind with an approximately 40 kilodalton protein found on the surface of ovarian cancer and other cells (Chang et al., 1996, Proc. Natl. Acad. Sci. USA 93(1):136-140). That protein, designated mesothelin, is derived from a larger (approximately 69 kilodalton) precursor protein for which the sequence is shown in Fig. 1. Scholler et al. (1999, Proc. Natl. Acad. Sci. USA 96(20):11531-11536) recognized that an antigen having an amino acid sequence highly similar to the amino terminal sequences of mesothelin and of megakaryocyte potentiating factor (MPF) occurs in the serum of many patients afflicted with ovarian carcinoma. Scholler proposed that this antigen could be a useful serum marker for diagnosing ovarian carcinoma.

A useful diagnostic test should not only be able to detect occurrence of ovarian cancer in a patient, it is also preferably inexpensive, non-invasive, and easy to use. Diagnostic tests that use blood or serum as a testing substrate require that blood be drawn from a patient to be screened, and sometimes require further separation, purification, or other treatment of the withdrawn blood sample. Drawing blood must be performed by a trained health care worker, is painful and invasive for the patient, requires specialized equipment and reagents for storage and transportation of blood samples, and carries the risk of transmission of blood-borne pathogens. By contrast, urine samples can be collected with little or no specialized equipment, do not require substantial oversight by a healthcare worker, are produced as a part of a patient's normal physiological processes, and generally are not significant sources of transmissible diseases. For these reasons, diagnostic testing using urine samples as a testing substrate can be preferable to comparable testing done with blood samples.

The present invention provides an ovarian cancer diagnostic test that can be performed using urine samples and that can be used to detect ovarian cancer at an early phase of disease progression.

### BRIEF SUMMARY OF THE INVENTION

The invention relates to a method of diagnosing an ovarian cancer (i.e., one or more of an epithelial, stromal, and germ cell ovarian cancer) in a woman. The method comprises assessing occurrence of a mesothelin/megak-aryocyte potentiating factor family (MMPFF) peptide in urine obtained from the woman. Occurrence of the MMPFF peptide in the urine is an indication that the woman is afflicted with an ovarian cancer.

In one embodiment, the MMPFF peptide is assessed in the woman's urine by contacting the urine (or centrifuged urine) with a first antibody that binds specifically with the MMPFF peptide. By assessing whether the MMPFF peptide has bound with the first antibody, occurrence of the MMPFF peptide in the woman's urine can be assessed. For example, the first antibody can be bound to a substrate, such as a plastic multi-well plate of the type adapted for automated analysis in a robotic apparatus. Binding of the MMPFF peptide and the first antibody can, for example, be assessed by contacting the first antibody with a second antibody that binds specifically with the MMPFF peptide and assessing co-localization of the first and second antibodies. The second antibody can be detectably labeled, such as with a compound selected from the group consisting of an enzyme, a radionuclide, a fluorophore, and a chromophore.

By way of example, the method can comprise contacting a plate having an anti-MMPFF peptide 'capture' antibody bound thereto with urine obtained from a woman. The urine can, optionally, be incubated with the plate for a period such as 10 minutes or an hour. The plate can then be contacted with a biotinylated second antibody that binds with an epitope of the MMPFF peptide than does the capture antibody. A streptavidin-linked enzyme can be bound to the biotinylated antibody to enable its detection in the presence of a chromogenic substrate (e.g., 3,3',5,5'-tetramethylbenzidine, which is a chromogenic substrate of horseradish peroxidase). Of course, the second antibody could instead be fluorescently labeled, radiolabeled, or otherwise detectably labeled.

In another embodiment of the method of diagnosing ovarian cancer, the first antibody can be contacted with a labeled substrate thereof contacting the first antibody with the urine. By comparing the amount of labeled ligand that binds with the first antibody that has been contacted with the urine with the amount of labeled ligand that binds with an equivalent amount of the first antibody that has not been contacted with the urine, one can assess how much of MMPFF from the urine has bound with the first antibody.

The kits and methods described herein can be used to detect a variety of MMPFF peptides, including naturally occurring MMPFF peptides (i.e., both full length proteins such as mesothelin and fragments of such proteins that are naturally produced in the bodies of ovarian cancer patients) and synthetically produced MMPFF peptides. The amino acid sequence of the MMPFF peptide should comprise at least 10 (20, 50, or 200) consecutive residues of a sequence selected from the group consisting of SEQ ID NOs: 1-5. Alternatively, the MMPFF peptide can comprise a portion of at least 20 consecutive amino acid residues, wherein the amino acid sequence of the portion is at least 90% (or 95%) identical to 20 consecutive residues of a sequence selected from the group consisting of SEQ ID NOs: 1-5.

The kits and methods disclosed herein can be used in conjunction with known or hereafter developed kits and methods for assessing occurrence (e.g., in urine or serum) other ovarian cancer markers. By way of example, ovarian cancer markers known to occur in urine include urinary gonadotropin peptide, cysteine proteinase, neopterin, and tumor-associated trypsin inhibitor. Known serum ovarian cancer markers include CA 125, carcinoembryonic antigen, vascular endothelial growth factor, tumor-associated trypsin inhibitor, a CD44 splice variant, anti-malignin antibody, lactate dehydrogenase, alpha-hydroxybutyrate dehydrogenase, CA19-9, tissue polypeptide antigen, and sialyl TN. CA 125 is known to be a surrogate marker for ovarian cancer occurrence and progression. Assessment of occurrence of MMPFF peptide in a woman's urine can likewise be performed in conjunction with assessment of the MMPFF peptide in the woman's serum.

Substantially the same kits and methods can be used to assess the likelihood that a woman will develop an ovarian cancer. That is, occurrence of an MMPFF peptide in urine obtained from a woman is an indication that the woman is more likely to develop an ovarian cancer than an otherwise identical woman in whose urine the MMPFF does not occur.

Substantially the same kits and methods can also be used to assess the efficacy of a therapy for an ovarian cancer in a woman. The amount of an MMPFF peptide in urine obtained from the woman following the therapy can be compared with the amount of the MMPFF in her urine before the therapy. A lower amount of the MMPFF peptide in the urine following the therapy is an indication that the therapy is efficacious. Similarly, a greater amount of the MMPFF peptide in the urine following the therapy is an indication that the therapy is not efficacious. Such kits and methods can be used to compare the efficacy of various therapeutic agents or regimens.

In another aspect, the invention relates to a kit for assessing occurrence of an MMPFF peptide in urine obtained from a woman. The kit comprises a first agent (e.g., an antibody) for binding the MMPFF peptide and an instructional material that describes contacting urine with the first agent. The format of the kit is not critical - many standard formats can be used, such as ELISA, latex bead aggregation, and surface plasmon resonance formats. The kit can include an MMPFF peptide for use as a positive control. Of course, the kit can also include reagents for assessing other known markers of ovarian cancer.

### BRIEF SUMMARY OF THE SEVERAL VIEWS OF THE DRAWINGS

In all figures comprising amino acid sequences, standard single-letter amino acid codes are used to represent amino acid residues.

Figure 1 is the amino acid sequence (SEQ ID NO: 1) of mesothelin precursor protein. This sequence is the same as that listed in GENBANK® accession number 2207386A and reported in Chang et al., 1996, Proc. Natl. Acad. Sci. USA 93(1):136-140.

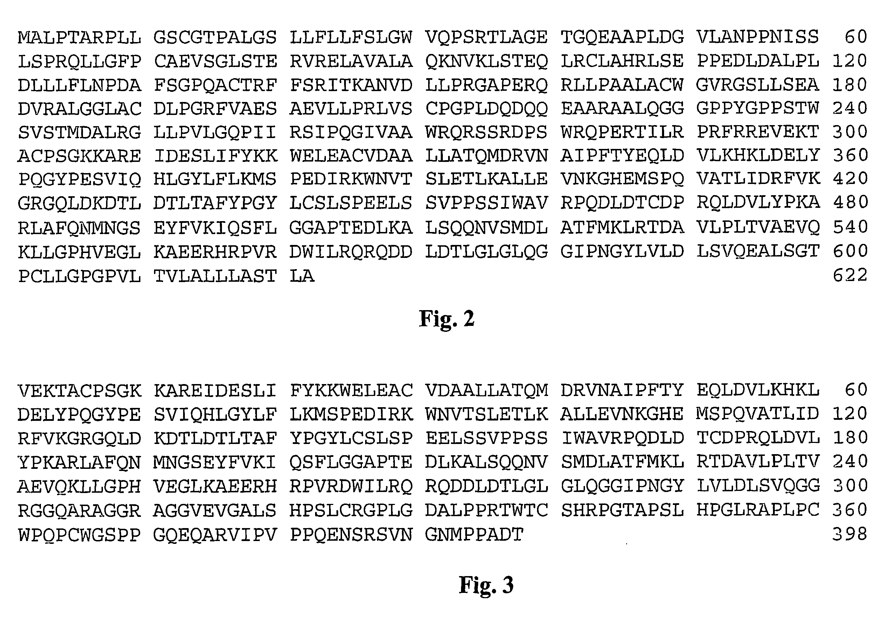

Figure 2 is the amino acid sequence (SEQ ID NO: 2) of megakaryocyte potentiating factor (MPF) precursor protein. This sequence is the same as that listed in GENBANK® accession number NP_005814 and reported in Yamaguchi et al., 1994, J. Biol. Chem. 269(2):805-808.

Figure 3 is a partial amino acid sequence (SEQ ID NO: 3) of a soluble variant form of MPF precursor protein listed in GENBANK® accession number AF180951 and reported in Scholler et al., 1999, Proc. Natl. Acad. Sci. USA 96(20):11531-11536.

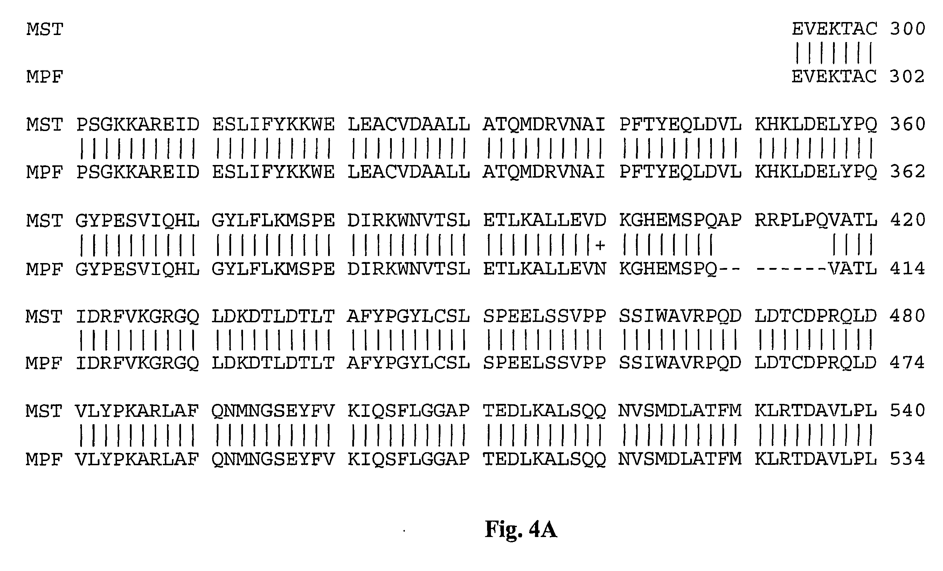

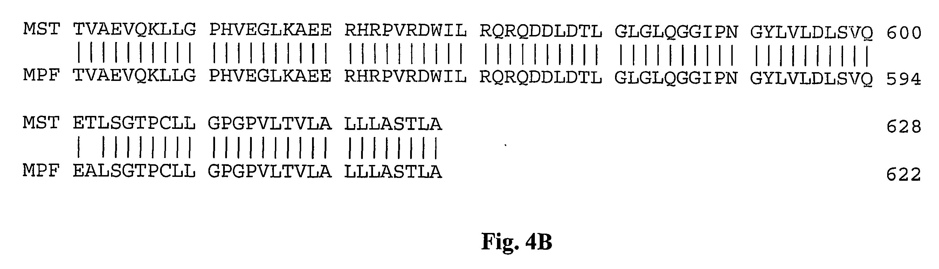

Figure 4, comprising Figures 4A and 4B, is a comparison of residues 294-628 of SEQ ID NO: 1 and a nearly identical portion of SEQ ID NO: 2. Residue numbers are listed in the right margin. "MST" means mesothelin. "MPF" means megakaryocyte potentiating factor. In this alignment, identical amino acid residues are indicated by a vertical line, a conservative amino acid substitution is indicated by a cross (+), and dashes (-) indicate a gap inserted into SEQ ID NO: 2 for the purpose of aligning the sequences.

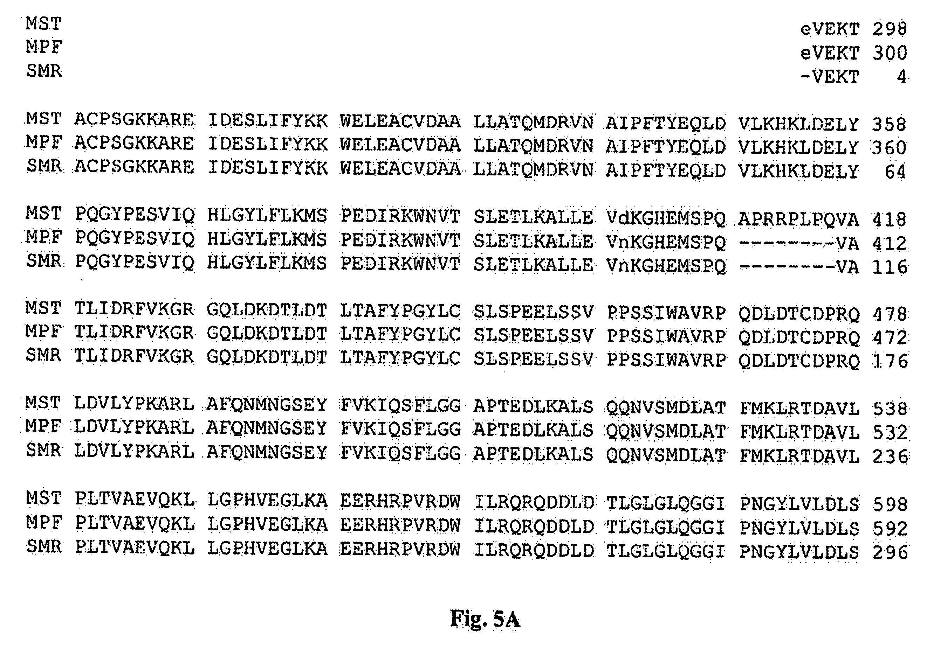

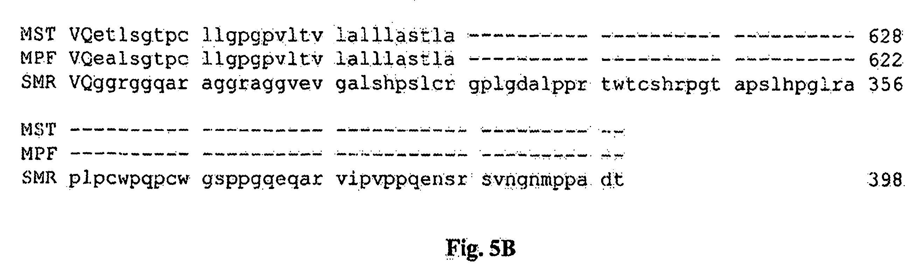

Figure 5, comprising Figures 5A and 5B, is a comparison of similar portions of SEQ ID NOs: 1-3. Residue numbers are listed in the right margin. "MST" means mesothelin. "MPF" means megakaryocyte potentiating factor. "SMR" means the soluble variant form of MPF for which the sequence is listed in Fig. 3. In this alignment, residues that are identical in all three sequences are represented by upper case single-letter codes, residues that are not identical in all three sequences are represented by lower case single-letter codes, and gaps inserted for the purpose of aligning the sequences are represented by dashes (-).

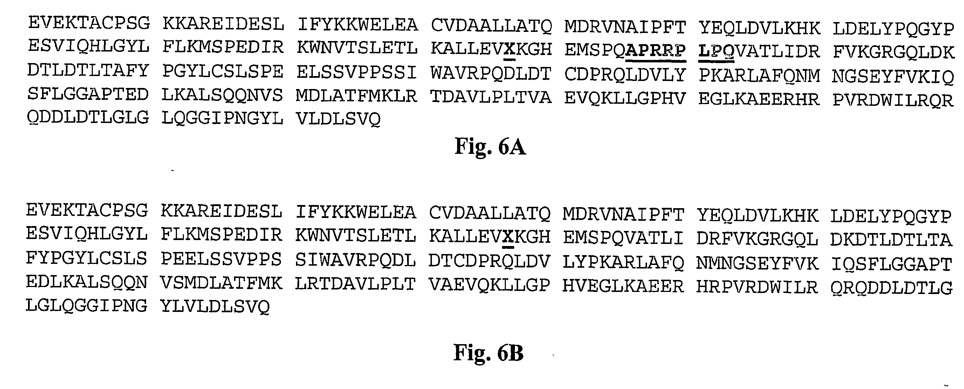

Figure 6 comprises Figures 6A and 6B. Figure 6A is an amino acid sequence (SEQ ID NO: 4) that includes preferred epitopes for generation of antibodies described herein. Figure 6B is another amino acid sequence (SEQ ID NO: 5) that includes preferred epitopes for generation of antibodies described herein. In each of SEQ ID NOs: 4 and 5, the underlined, bold X represents any amino acid residue, preferably being either aspartate or asparagine. Other underlined, bold residues in Figure 6A represent residues that are not present in the sequence shown in Figure 6B.

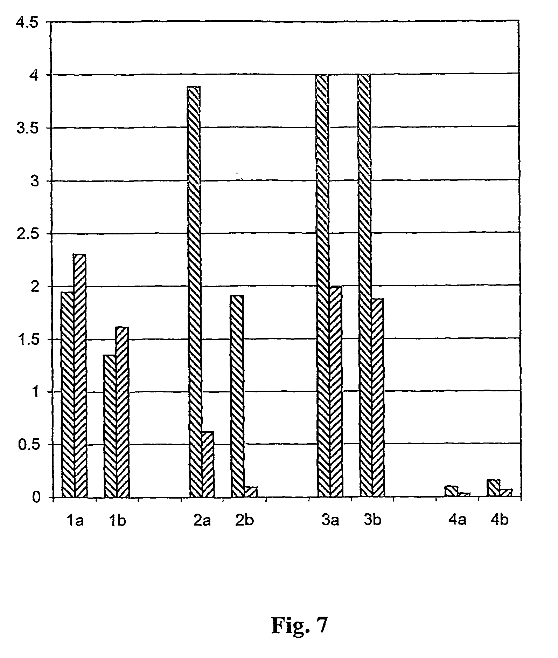

Figure 7 is a bar graph that indicates serum levels of CA125II antigen (bars with stripes slanting downward from left to right) and urine levels of an MMPFF peptide (bars with stripes slanting upward from left to right), assayed as disclosed herein. Assays were performed for four patients, designated 1-4, both at the time of ovarian cancer diagnosis ("a") and approximately one month after the onset of therapy ("b").

### DETAILED DESCRIPTION OF THE INVENTION

The invention relates to the discovery that one or more polypeptides having significant amino acid sequence similarity with a family of proteins that includes mesothelin, megakaryocyte potentiating factor (MPF), and a soluble variant of MPF occurs in the urine of women afflicted with ovarian cancer. These urine polypeptides bind specifically with antibodies that are raised against proteins of that family. Occurrence of these polypeptides in a patient's urine is diagnostic that the patient is afflicted with ovarian cancer. The amount of the polypeptides decreases with effective treatment of the ovarian cancer. Thus, the urine polypeptides can also be used to assess the efficacy of anti-ovarian cancer therapy.

Definitions

As used herein, each of the following terms has the meaning associated with it in this section.

A mesothelin/megakaryocyte potentiating factor family peptide ("MMPFF peptide") is a polypeptide that has an amino acid sequence that either (i) comprises at least 10 consecutive residues of a sequence selected from the group consisting of SEQ ID Noes : 1-5 or (ii) comprises a portion of at least 20 consecutive amino acid residues wherein the amino acid sequence of the portion is at least 90% (preferably at least 95% or 100%) identical to 20 consecutive residues of a sequence selected from the group consisting of SEQ ID NOs: 1-5.

An "antibody" refers collectively and individually to immunoglobulin molecules and immunologically active portions of immunoglobulin molecules, i.e., molecules that contain an antigen binding site which specifically binds an MMPFF peptide. A molecule which specifically binds with an MMPFF peptide is a molecule which binds the peptide, but does not substantially bind other molecules in a sample (e.g., a urine sample) which naturally contains the polypeptide. Examples of immunologically active portions of immunoglobulin molecules include F(ab) and F(ab')₂ fragments which can be generated by treating the antibody with an enzyme such as papain or pepsin, respectively. Either or both of polyclonal and monoclonal antibodies can be used in the methods and kits described herein.

The term "monoclonal antibody" refers to a population of antibody molecules that contain only one species of an antigen binding site capable of immunoreacting with a particular epitope.

The term "labeled," with regard to an antibody or a peptide, includes direct labeling of the peptide or antibody by coupling (i.e., physically linking) a detectable substance to the peptide or antibody, as well as indirect labeling of the peptide or antibody by coupling it with another reagent that is directly labeled. Examples of indirect labeling include detection of a primary antibody using a fluorescently labeled secondary antibody and end-labeling of a peptide with biotin such that it can be detected with fluorescently labeled streptavidin.

An "instructional material" is a publication, a recording, a diagram, or any other medium of expression which can be used to communicate how to use a kit described herein, information for interpreting occurrence of an MMPFF in urine as it relates to an ovarian cancer, or both. The instructional material of the kit of the invention can, for example, be affixed to a container which contains a kit of the invention or be shipped together with a container which contains the kit. Alternatively, the instructional material can be shipped separately from the container with the intention that the instructional material and the kit be used cooperatively by the recipient.

Detailed Description

It has been discovered that one or more polypeptides having significant amino acid sequence similarity with a family of proteins (the mesothelin/megakaryocyte potentiating factor family; "MMPFF") occurs in the urine of women afflicted with ovarian cancer. These urine peptides can be detected, for example with an antibody raised against a protein of the MMPFF. Occurrence of such peptides in a patient's urine is an indication that the patient is afflicted with ovarian cancer. Owing to the variety of ovarian cancer types, the genetic variability among individuals, and other factors, MMPFF peptides will likely not appear in the urine of all patients afflicted with ovarian cancer, and the peptides will likely appear in differing amounts in individuals afflicted with ovarian cancers of substantially the same type. Even taking these variations into account, occurrence of one or more MMPFF peptides in the urine of a woman is an indication that the woman is afflicted with ovarian cancer.

Advantageously, occurrence of MMPFF peptides in the urine of women afflicted with ovarian cancer does not appear to depend on the stage by which the ovarian cancer is characterized. For this reason, urinary occurrence of MMPFF peptides can be used to diagnose ovarian cancer at an early stage, facilitating efficacious treatment of the cancer before it has reached a phase of the disease at which therapy is futile or nearly so.

Another advantage of the kits and methods disclosed herein is that the amount and concentration of MMPFF peptide in the urine of an ovarian cancer patient is indicative of the state of the tumor. For this reason, assessment of urinary MMPFF peptide occurrence can be used to assess the efficacy of an ovarian cancer therapeutic regimen. To the extent the regimen induces tumor shrinkage or regression, urinary concentration of MMPFF peptide can be expected to decrease. Similarly, gradual or unimpeded increase in urinary MMPFF peptide concentration during ovarian cancer therapy is indicative of a therapeutic regimen that is not inhibiting tumor growth.

The kits and methods described herein can be used to assess occurrence or progression of various ovarian cancer types. In an important embodiment, the kits and methods are used to assess occurrence or progression of an epithelial ovarian cancer, which are among the most common ovarian cancers. In other embodiments, the kits and methods are used to assess occurrence of progression of stromal ovarian tumors or germ cell ovarian tumors.

Diagnostic Methods

The invention includes a method of diagnosing an ovarian cancer in a woman. The method comprises assessing occurrence of a mesothelin/megakaryocyte potentiating factor family (MMPFF) peptide in urine obtained from the woman. Occurrence of the MMPFF peptide in the urine is an indication that the woman is afflicted with an ovarian cancer. Ovarian cancers of various type (e.g., epithelial ovarian cancer), grade, and stage can be diagnosed using this method. The method can also be used to diagnose ovarian cancers characterized by various cell types (e.g., serous, mucinous, endometrioid, clear, and non-differentiated ovarian tumors).

The method used to assess occurrence of the MMPFF peptide in the woman's urine is not critical. Any method that can be used to assess whether the peptide is present or absent is suitable. Methods capable of assessing the quantity (e.g., concentration) of the MMPFF peptide in the urine are preferable in some embodiments, particularly when assessments made over a period of time are to be compared (e.g., as when assessing the efficacy of a regimen of anti-cancer therapy).

In one embodiment, occurrence of the MMPFF peptide in the patient's urine is assessed by contacting the urine with an antibody that binds specifically with the MMPFF peptide and assessing whether the MMPFF peptide has bound with the first antibody.

Numerous MMPFF-binding antibodies are known in the art. For example, the antibody described in U.S. Patent No. 5,320,956 and secreted by the hybridoma deposited with the ATCC as accession no. HB 10570 can be used. That antibody binds specifically with an approximately 40 kilodalton portion of mesothelin protein, as described by Chang et al. (1996, Proc. Natl. Acad. Sci. USA 93(1):136-140). Other suitable antibodies have been described by Scholler et al. (1999, Proc. Natl., Acad. Sci. USA 96(20):11531-11536) and in PCT publication number WO 00/50900, wherein such antibodies are designated OV569, 4H3, 3G3, and 1A6.

In diagnostic kits and methods that use two anti-MMPFF peptide antibodies, the two antibodies preferably do not compete to bind the same epitope. By way of example, the antibody designated OV569 by Scholler et al. binds at an epitope which is independent of the epitopes bound by the antibodies designated 4H3, 3G3, and 1A6. Thus, for example, OV569 can be bound to a substrate and used as a 'capture' antibody to bind MMPFP peptide present in a patient's urine under appropriate antibody-binding conditions, and one of antibodies 4H3, 3G3, and 1A6 can be detectably labeled and contacted with the 'capture' antibody after contacting the 'capture' antibody with the patient's urine. Binding of the detectably labeled antibody with the 'capture' antibody indicates that the MMPFF peptide was present in the patient's urine. Analysis of the amount of detectable label co-localized with the 'capture' antibody can indicate the quantity of the peptide that was present.

As described herein, portions of MMPFF proteins share significant amino acid sequence homology. Figs. 1-3 show at least portions of the amino acid sequences of three MMPFF proteins. Figure 4 and 5 show alignments of those sequences, indicating the regions of greatest sequence homology among these proteins. From these figures, it is apparent that the sequences shown in Figs. 6A and 6B (SEQ ID NOs: 4 and 5, respectively) represent useful portions of MMPFF peptides. Antibodies raised against all or a portion (e.g., 10, 20, 50, or 200 consecutive residues) of either of these sequences can be expected to bind specifically with a broad range of MMPFF peptides, including those that occur in the urine of ovarian cancer patients.

An MMPFF (e.g., one described herein or in the prior art), or a fragment thereof, can be used as an immunogen to generate antibodies using standard techniques for polyclonal and monoclonal antibody preparation. A fragment of a full-length MMPFF, when used as an immunogen, should comprise at least 10 (preferably 12, 15, 20, 50, 100, or 20 or more) amino acid residues of the amino acid sequence of any of SEQ ID NOs: 1-5 or the amino acid sequence of another MMPFF member.

Appropriate MMPFF peptides for generation of antibodies useful in the kits and methods described herein comprise a portion of at least 20 consecutive amino acid residues that is at least 90% (preferably at least 95% or 100%) identical to 20 consecutive residues of a sequence selected from the group consisting of SEQ ID NOs: 1-5 (preferably either SEQ ID NO: 4 or SEQ ID NO: 5).

Methods of generating antibodies are well known and only briefly summarized here. An immunogen typically is used to prepare antibodies by immunizing a suitable (i.e., immunocompetent) subject such as a rabbit, goat, mouse, or other mammal or vertebrate. An appropriate immunogenic preparation can contain, for example, recombinantly-expressed or chemically-synthesized polypeptide. The preparation can further include an adjuvant, such as Freund's complete or incomplete adjuvant, or a similar immunostimulatory agent.

Polyclonal antibodies can be prepared as described above by immunizing a suitable subj ect with a polypeptide of the invention as an immunogen. The antibody titer in the immunized subject can be monitored over time by standard techniques, such as with an enzyme linked immunosorbent assay (ELISA) using immobilized polypeptide. If desired, the antibody molecules can be harvested or isolated from the subject (e.g., from the blood or serum of the subject) and further purified by well-known techniques, such as protein A chromatography to obtain the IgG fraction.

At an appropriate time after immunization, e.g., when the specific antibody titers are highest, antibody-producing cells can be obtained from the subject and used to prepare monoclonal antibodies by standard techniques, such as the hybridoma technique originally described by Kohler and Milstein (1975) Nature 256:495-497, the human B cell hybridoma technique (Kozbor et al. (1983) Immunol. Today 4:72), the EBV-hybridoma technique (Cole et al. (1985), Monoclonal Antibodies and Cancer Therapy, Alan R. Liss, Inc., pp. 77-96) or trioma techniques. The technology for producing hybridomas is well known (see generally Current Protocols in Immunology (1994) Coligan et al. (Eds.) John Wiley & Sons, Inc., New York, NY). Hybridoma cells producing a monoclonal antibody of the invention are detected by screening the hybridoma culture supernatants for antibodies that bind the polypeptide of interest, e.g., using a standard ELISA assay.

Alternative to preparing monoclonal antibody-secreting hybridomas, a monoclonal antibody directed against a polypeptide of the invention can be identified and isolated by screening a recombinant combinatorial immunoglobulin library (e.g., an antibody phage display library) with the polypeptide of interest. Kits for generating and screening phage display libraries are commercially available (e.g., the Pharmacia Recombinant Phage Antibody System, Catalog No. 27-9400-01; and the Stratagene SURFZAP™ Phage Display Kit, Catalog No. 240612). Additionally, examples of methods and reagents particularly amenable for use in generating and screening antibody display library can be found in, for example, U.S. Patent No. 5,223,409; PCT Publication No. WO 92/18619; PCT Publication No. WO 91/17271; PCT Publication No. WO 92/20791; PCT Publication No. WO 92/15679; PCT Publication No. WO 93/01288; PCT Publication No. WO 92/01047; PCT Publication No. WO 92/09690; PCT Publication No. WO 90/02809; Fuchs et al. (1991) Bio/Technology 9:1370-1372; Hay et al. (1992) Hum. Antibod. Hybridomas 3:81-85; Huse et al. (1989) Science 246:1275-1281; Griffiths et al. (1993) EMBO J. 12:725-734.

Recombinant antibodies, such as chimeric and humanized monoclonal antibodies, comprising both human and non-human portions, which can be made using standard recombinant DNA techniques, are within the scope of the invention. Such chimeric and humanized monoclonal antibodies can be produced by recombinant DNA techniques known in the art, for example using methods described in PCT Publication No. WO 87/02671; European Patent Application 184,187; European Patent Application 171,496; European Patent Application 173,494; PCT Publication No. WO 86/01533; U.S. Patent No. 4,816,567; European Patent Application 125,023; Better et al. (1988) Science 240:1041-1043; Liu et al. (1987) Proc. Natl. Acad. Sci. USA 84:3439-3443; Liu et al. (1987) J. Immunol. 19:3521-3526; Sun et al. (1987) Proc. Natl. Acad. Sci. USA 84:214-218; Nishimura et al. (1987) Cancer Res. 47:999-1005; Wood et al. (1985) Nature 314:446-449; and Shaw et al. (1988) J. Natl. Cancer Inst. 80:1553-1559); Morrison (1985) Science 229:1202-1207; Oi et al. (1986) Bio/Techniques 4:214; U.S. Patent 5,225,539; Jones et al. (1986) Nature 321:552-525; Verhoeyan et al. (1988) Science 239:1534; and Beidler et al. (1988) J. Immunol. 141:4053-4060.

In embodiments in which an anti-MMPFF peptide antibody is contacted with urine obtained from a patient, the urine can be used in its naturally-expressed state or it can be partially purified or clarified prior to use. For example, urine can be centrifuged using standard methods to substantially remove any sediment therefrom prior to contacting the urine with the antibody. Alternatively, the urine can be filtered or ultrafiltered to reduce its volume or to remove large contaminants. Because MMPFF peptides that occur in urine can be expected to be polypeptides having molecular weights in the range 10,000 - 100,000 Daltons, selection of an appropriate filtration/ultrafiltration is necessary to prevent removal of the MMPFF peptide prior to its detection. By way of example, a relatively large pore filter (e.g., one that permits passage of globular particles having molecular weights of 250,000 to 1 million Daltons can be used to remove particulate material from urine and an ultrafiltration device equipped with a membrane that excludes passage of proteins having molecular weights greater than about 5,000 Daltons can be used to concentrate the filtrate. In this example, occurrence of the MMPFF can be assessed in the retentate in the ultrafiltration device.

Because MMPFF peptides are often relatively abundant in urine of ovarian cancer patients and because many of the methods (e.g., the immunological methods) described herein are relatively sensitive, concentration of urine is usually not necessary to detect occurrence of MMPFF peptides in urine of ovarian cancer patients. In situations in which clarification of urine is desired, centrifugation can be preferable, since the potential that MMPFF peptides present in the urine will become bound with or enmeshed within the filter medium is not present.

One preferred method for assessing occurrence of an MMPFF peptide in urine of a woman is the procedure commonly known as a "sandwich ELISA" assay. (ELISA is an abbreviation for enzyme-linked immunosorbent assay.) In this method, an antibody is bound to a substrate, such as a glass bead or the bottom surface of a plastic multi-well assay plate. This antibody is designated a 'capture' antibody. Raw, clarified, or purified urine from the woman is contacted with the substrate under conditions (e.g., low concentrations of salt and detergents and non-protein-denaturing conditions), so that any MMPFF peptide present in the urine binds specifically with the capture antibody. The substrate is optionally rinsed with a fluid that does not comprise the MMPFF peptide to remove residual urine and any non-bound MMPFF. The substrate is then contacted with a second antibody that specifically binds with the MMPFF peptide at an epitope independent of the epitope at which the capture antibody binds. The second antibody is either detectably labeled or linked with either a ligand or a receptor of the ligand. Either way, binding of the second antibody is detected (and, optionally, quantitated) after rinsing the substrate with a fluid that does not comprise the second antibody. Detection of the second antibody is indicative of the presence in the urine of the MMPFF peptide. If the amount of the second antibody is quantitated, then the amount of the MMPFF peptide in the urine can be quantitated, for example, by comparison of the amount of second antibody detected with measurements made using control samples containing known amounts of the MMPFF.

Detection an antibody can be facilitated by coupling the antibody to a detectable substance. Examples of detectable substances include various enzymes, prosthetic groups, fluorescent materials, luminescent materials, bioluminescent materials, and radioactive materials. Examples of suitable enzymes include horseradish peroxidase, alkaline phosphatase, beta-galactosidase, or acetylcholinesterase; examples of suitable prosthetic group complexes include streptavidin / biotin and avidin / biotin; examples of suitable fluorescent materials include umbelliferone, fluorescein, fluorescein isothiocyanate, rhodamine, dichlorotriazinylamine fluorescein, dansyl chloride or phycoerythrin; an example of a luminescent material includes luminol; examples of bioluminescent materials include luciferase, luciferin, and aequorin, and examples of suitable radioactive material include ¹²⁵I, ¹³¹I, ³⁵S or ³H.

Use of standardized assay apparatus permits automation of the method described herein. For example, many different standardized assay containers are known, such as 24-, 48-, 96-, and 384-well plastic plates. Where these containers are adapted to fit a robotic apparatus, automated analysis of samples can be achieved, permitting high-throughput screening of many samples in a relatively short time. Automated assay apparatus and containers adapted for their use in computer-controlled assays are known in the art and readily adapted for the kits and methods described herein.

Another type of immunological assay that can be used to assess occurrence of an MMPFF peptide in urine obtained from a woman is commonly designated a 'competition' assay. In this assay, an antibody that binds specifically with the MMPFF peptide is fixed to a substrate. Raw, clarified, or purified urine is contacted with the substrate (preferably for a period of minutes or hours), so that any MMPFF peptide present in the urine binds with the antibody. A labeled ligand (e.g., a labeled version of the same MMPFF peptide or another MMPFF peptide) that binds specifically with the antibody is then contacted with the substrate (preferably for a period of minutes or hours). The amount of labeled ligand bound with the substrate is assessed after rinsing the substrate with a fluid that does not comprise the label or the labeled ligand. The amount of label bound with the substrate is compared with the amount of label bound with an otherwise identically treated substrate that is not contacted with the patient's urine. The difference between the two amounts of bound label represents the amount of MMPFF peptide bound to the substrate, and is indicative of the amount of the MMPFF peptide that was present in the urine sample applied to the substrate.

When an immunological competition assay is used, the labeled ligand of the antibody bound with the substrate should be as nearly identical to the MMPFF peptide known or expected to be present in the patient's urine as possible. For example, the ligand and the MMPFF peptide should have or comprise the same amino acid sequence. Similarly, if the MMPFF peptide that may occur in the patient's urine is expected to by glycosylated, then the ligand should be glycosylated in the same way, at the same position, and to the same extent. In this way, affinity differences of the antibody for the ligand and for the MMPFF peptide can be minimized and the accuracy of the competition assay can be improved.

The kits and methods described herein can be used alone to assess occurrence of an MMPFF peptide in urine of a human patient, and such occurrence is indicative that the woman is afflicted with ovarian cancer. However, a greater number of ovarian cancers can be detected and greater confidence in the diagnosis of ovarian cancer can be achieved if occurrence of more than one marker of ovarian cancer is assessed in the woman. Any additional ovarian cancer marker(s) can be a marker normally found in urine, a marker normally found in blood, or a marker normally found in both urine an blood. By way of example, MMPFF peptides are normally found in the serum of patients afflicted with ovarian cancer (Scholler et al., 1999, Proc. Natl. Acad. Sci. USA 96(20):11531-11536). Thus, the kits and methods described herein for assessing occurrence of MMPFF peptides in urine can be used in conjunction with kits and methods of detecting the same or other MMPFF peptides in serum.

Other ovarian cancer markers known to occur in urine include urinary gonadotropin peptide (UGP; Schutter et al., 1999, Anticancer Res. 19(6C):5551-5557), cysteine proteinase (UCP; Gao et al., 1996, Hua Xi Yi Ke Da Xue Xue Bao 27(3):291-294), neopterin (Szarka et al., 1988, Acta Chir. Hung. 29(4):359-364), and tumor-associated trypsin inhibitor (TATI; Medl et al., 1995, Br. J. Cancer 71(5):1051-1054; Halila et al., 1988, Br. J. Cancer 57(3):304-307).

Other ovarian cancer markers known to occur in serum include CA 125 (Halila et al., 1987, Br. J. Cancer 56(2):153-156), carcinoembryonic antigen (CEA; Juweid et al., 1997, Gynecol. Oncol. 67(3):259-271), vascular endothelial growth factor (VEGF; Candido Dos Reis et al., 2002, Gynecol. Obstet. Invest. 54(3):132-136), tumor-associated trypsin inhibitor (TATI; Medl et al., 1995, Br. J. Cancer 71(5):1051-1054; Halila et al., 1988, Br. J. Cancer 57(3):304-307), CD44 splice variants (Uhl-Steidl et al., 1995, Oncology 52(5):400-406), anti-malignin antibody (Bogoch et al., 1991, Lancet 337:927), lactate dehydrogenase (LDH), alpha-hydroxybutyrate dehydrogenase (aHBDH), CA19-9, tissue polypeptide antigen (TPA), and sialyl TN (STN; Kudoh et al., 1999, Gynecol. Obstet. Invest. 47(1);52-57). This invention includes kits and methods for assessing any combination of these known markers in blood and/or urine, so long as the kits and methods include assessing occurrence of at least MMPFF peptide in urine.

The kits and methods described herein for assessing occurrence of a MMPFF peptide in urine obtained from a woman can be used to assess the likelihood that the woman will develop an ovarian cancer. Detection of occurrence of an MMPFF peptide in a woman's urine is an indication that the woman is more likely to develop an ovarian cancer than an otherwise identical woman in whose urine the MMPFF does not occur. It is recognized that the difference between being afflicted with an ovarian tumor, the earliest stages of tumor growth, the onset of tumorigenesis, and enhanced susceptibility to ovarian tumorigenesis may be substantially indistinguishable. Nonetheless, the outcome of each of these processes is development of an ovarian tumor to the detriment of the woman's health. For this reason, assessment of any of these states using the kits and methods disclosed herein is useful.

Anti-MMPFF peptide antibodies can be used diagnostically or prognostically to monitor MMPFF levels in urine as part of a clinical testing procedure, e.g., to, for example, determine the efficacy of a given ovarian cancer treatment regimen.

The kits and methods described herein can be used to determine whether an agent (e.g., an agonist, antagonist, peptidomimetic, protein, peptide, nucleic acid, small molecule, or other drug candidate) can be administered to a subject in order to alleviate, inhibit, reverse, or prevent ovarian cancer. For example, such methods can be used to determine whether an ovarian cancer patient can be effectively treated using a specific agent or class of agents.

Kits for Assessing Occurrence of Urinary MMPFF Peptides

The invention also encompasses kits for detecting occurrence of an MMPFF in a urine sample obtained from a mammal (e.g., a woman). The kit comprises a first agent for binding the MMPFF peptide, a second agent for assessing binding of the MMPFF peptide with the agent, and an instructional material that describes contacting urine with the first agent. These kits can be used to determine if a subject is suffering from or is at increased risk of developing ovarian cancer. For example, the kit can comprise a labeled compound or agent capable of detecting the MMPFF peptide in a urine sample. The kit can also, or alternatively, contain means for determining the amount of the MMPFF peptide in the sample. Kits can include instructions for assessing whether the tested subject is suffering from or is at risk of developing ovarian cancer if the amount of the MMPFF peptide is above or below a normal level (e.g., an absorbance measurement of at least 0.2 at 450 nanometers using the methodology set forth in Example 1).

For antibody-based kits, the kit can comprise, for example: (1) a first antibody (e.g., attached to a solid support) which specifically binds with an MMPFF peptide and, optionally, (2) a second, different antibody which specifically binds with either the MMPFF peptide (e.g., at a epitope distinct from the epitope at which the first antibody binds) or the first antibody and is conjugated with a detectable agent. Alternatively, the kit can comprise the first antibody and a labeled ligand of the first antibody. After contacting the first antibody with a woman's urine sample, binding of the labeled ligand with the first antibody can be assessed in a competition-type assay, as described herein.

The kits can further comprise reagents and/or instructions for assessing occurrence in urine or serum of the woman another marker indicative of occurrence of ovarian cancer in the woman.

Examples

The invention is now described with reference to the following Examples. These Examples are provided for the purpose of illustration only, and the invention is not limited to these Examples, but rather encompasses all variations which are evident as a result of the teaching provided herein.

Example 1

Sandwich ELISA Assay for Assessing MMPFF in a Sample

A typical assay used to detect an MMPFF in a urine sample is now described. Assays were performed in a 96-well clear, flat-bottom microtiter plate that had been previously coated with monoclonal antibody 4H3 (as described in Scholler et al., 1999, Proc. Natl. Acad. Sci. USA 96(12):111531-11536 and in PCT publication WO 00/50900). The bottom of the wells were coated with the antibody by adding to individual wells 50 microliters of a suspension comprising 3 micrograms of antibody per milliliter in carbonate-bicarbonate buffer (pH 9.6). The plate was swirled to coat the bottom surface of the wells and then permitted to dry overnight at 4°C. Each well was blocked by filling it with a 3% (w/v) suspension of bovine serum albumin (BSA) and maintaining the filled wells for 2 hours at room temperature, after which time the BSA suspension was removed from the wells.

Standard curves were prepared using a fusion protein (as described in Scholler et al., 1999, Proc. Natl. Acad. Sci. USA 96(20):11531-11536) comprising a portion of the MPF amino acid sequence (i.e., the 44 kDa membrane-bound domain) fused to an immunoglobulin (Ig) constant region using a described vector encoding a human Ig sequence (as described in PCT publication number WO 00/50900 and in Hollenbaugh et al., 1995, J. Immunol. Meth. 188:1-7). This fusion protein was designated hIgG. For the standard curve, the fusion protein was assessed at concentrations of 1.0 microgram per milliliter, 0.1 microgram per milliliter, and four serial 5-fold dilutions thereof (i.e., 1:5, 1:25, 1:125, and 1:625). The diluent used was a 7% (w/v) suspension of BSA.

To perform the assay, SO microliters of an hIgG fusion protein dilution, a control, or a urine sample, or a diluted urine sample was added to individual wells. The plate was incubated for 1 hour at room temperature and the wells were washed with tris-buffered saline (pH 7.8 to 8) containing 0.05% (v/v) TWEEN® 20 surfactant. 50 microliters of a 200 nanogram per milliliter suspension of biotinylated monoclonal antibody OV569 (as described in Scholler et al., 1999, Proc. Natl. Acad. Sci. USA 96(20):11531-11536 and in PCT publication number WO 00/50900) was added to each well. After incubating the plate for 1 hour at room temperature and thereafter washing each well with tris-buffered saline (pH 7.8 to 8) containing 0.05% (v/v) TWEEN® 20 surfactant, 50 microliters of a 1:5000 dilution of Streptavidin-horseradish peroxidase (obtained from Southern Biotechnology Associates, Inc., Birmingham, AL, Catalog no. 7100-05) at was added to each well, and the plate was incubated for 1 hour at room temperature. 50 Microliters of 3,3',5,5'-tetramethylbenzidine (TMB; a chromogenic substrate for horseradish peroxidase; obtained from Kirkegaard & Perry Laboratories, Inc., Gaithersburg, MD) was added to each well to generate signal. The plate was incubated for 15 minutes at room temperature before adding 50 microliters of TMB Stop solution (0.1 normal sulfuric acid; obtained from Kirkegaard & Perry Laboratories, Inc., Gaithersburg, MD) to halt the peroxidase reaction. Blue color generated by the action of the peroxidase on TMB was assessed using a Molecular Devices SpectraMax® Plus 3 84 spectrophotometric plate reader (model number 02523) to measure the absorbance at 450 nanometers.

Example 2

Detection of an MMPFF in Urine Samples Obtained from Ovarian Cancer Patients

Urine samples were obtained from 25 patients known to be afflicted with ovarian cancer. Individual urine samples were centrifuged for 15 minutes at 13,000 rpm (ca. 15,700 x g) to remove sediment. Individual 50 microliter aliquots of urine sample supernatants were tested using the assay described in Example 1.

Of the 25 samples that were tested, 22 of the samples registered an absorbance value at 450 nanometers that was greater than 0.2. These data indicate that use of a chromogenic sandwich ELISA-type assay for detecting an MMPFF in urine is a reliable way of detecting occurrence of ovarian cancer in many patients.

Example 3

Confirmation of MMPFF Signal in Urine Samples Obtained from Ovarian Cancer Patients

In order to rule out non-specific binding that may have mimicked signal corresponding to occurrence of MMPFF in a urine sample described in Example 2, urine samples for which relatively high absorbance values were obtained in Example 2 were selected. An assay plate was prepared as described in Example 1, except that the plate had not been coated with antibody 4H3. Individual urine samples were prepared and added to the plate as described in Example 2.

No signal above the background level was detected in any of the samples tested in this Example.

In a separate experiment, the urine samples described in Example 2 were tested using a plate prepared as described in Example 1. The 569 monoclonal antibody used in this experiment was not biotinylated, however. Otherwise, the assay was as described in Example 1. No signal above background level was detected for any of the samples tested.

In another separate experiment, the assay was performed as described in Example 1, except that a biotinylated antibody that has no relation to the MMPFF was used in place of biotinylated monoclonal antibody 569. Assays of the urine samples described in Example 2 by this method yielded no signal above background when tested in this manner.

These data indicate that the increased absorbance signal at 450 nanometers observed in the sandwich ELISA assay using urine obtained from ovarian cancer patients is a true response indicating the presence of the MMPFF peptide in the patients' urine.

Example 4

Urine samples obtained from 31 patients who had been diagnosed with bladder cancer, from 3 women who were afflicted with benign gynecological diseases, and from 22 urine samples obtained from normal, healthy adults were tested using the assay described in Example 1.

The data demonstrate that urine obtained from normal, healthy patients has very low levels of MMPFF peptide. As noted in Example 2, urine from 22 of 25 ovarian cancer patients (88%) exhibited significantly elevated MMPFF peptide levels. Urine samples obtained from bladder cancer patients (i.e., another genitourinary disease) exhibited elevated levels of MMPFF peptide in only 8 of the 31 samples. Elevated MMPFF peptide levels were not detected in the 3 urine samples obtained from women who were afflicted with benign gynecological diseases. Taken together, urine samples obtained from only 8 of 56 non-ovarian cancer patients exhibited enhanced MMPFF peptide levels, while 22 of 25 urine samples from ovarian cancer patients did. These data indicate that the specificity of this assay method for ovarian caner is 86%.

Example 5

Correlation of Urine MMPFF Peptide Levels with Stage of Ovarian Cancer

Information regarding the stage of their cancer was collected for each of the 25 ovarian cancer patients from whom urine samples were analyzed in Example 2. Patients having ovarian cancers for each of the four stages were represented. There was no apparent correlation between the stage of the cancer for the patient and the amount of MMPFF peptide detected in the patient's urine. An implication of this observation is that the urine MMPFF peptide assay method described herein can be used to assess occurrence of early stage ovarian cancer as effectively as late stage ovarian cancer.

Example 6

Effect of Ovarian Cancer Therapy on Urinary MMPFF Peptide Level

The effect of ovarian cancer treatment on 4 of the 25 ovarian cancer patients described in Example 2 on MMPFF peptide levels were assessed and compared with serum levels of the ovarian cancer tumor marker designated CA 125. CA 125 was assessed in serum samples obtained from each of the four patients before and after treatment, using an immunoassay substantially similar to the CA125II™ (Fujirebio Diagnostics, Inc., Malvern, PA) assay. Urine MMPFF peptide levels were also assessed in each of these four patients before and after treatment, using the methods described in Examples 1 and 2.

The change in urine MMPFF peptide level before and after treatment was approximately proportional to the change in serum CA 125 level in each of the four patients, as illustrated in Fig. 7. Because CA 125 is accepted in the art as a surrogate marker of ovarian cancer progression, these data demonstrate that a correlation exists between urinary MMPFF peptide levels and disease progression. These data confirm the clinical value of this test and its potential use as a non-invasive method of monitoring ovarian cancer therapy efficacy.

Example 7

Correlation Between Urinary and Serum Levels of MMPFF Peptide

Blood was drawn from each of the 25 patients who provided the urine samples assayed in Example 2. Serum MMPFF peptide levels were determined for those blood samples, using the protocol described in Example 1. Serum and urine samples were collected from individual patients on the same day.

MMPFF peptides could be detected in both urine and serum samples from all patients, albeit at very low levels in some instances. Using a cut-off value of 0.2 absorbance units (at 450 nanometers) for the test of urinary samples, 22 out of 25 urine samples (88%) tested positive for MMPFF. Using a cut-off value of 0.1 absorbance units (at 450 nanometers) for the test of serum samples, only 16 of the 25 (64%) tested positive for MMPFF. These data demonstrate that MMPFF peptide level is elevated in both the serum and urine of ovarian cancer patients. However, detection of MMPFF peptide in urine appears to be more sensitive and may therefore have greater diagnostic value.

Example 8

Assessment of Urine CA 125 Levels

CA 125 level was assessed in each of the 25 urine samples described in Example 2 using the CA125II™ test kit. None of the urine samples yielded results above the assay's background level. These results indicate that CA 125 is not present in the urine of these patients. These data also confirm that serum tumor markers such as CA 125 are not necessarily present in the urine of patients in whose serum they occur.

## Claims

1. A method of diagnosing an ovarian cancer in a woman, the method comprising assessing occurrence of a mesothelin/megakaryocyte potentiating factor family peptide in urine obtained from the woman, whereby occurrence of the MMPFF peptide in the urine is an indication that the woman is afflicted with an ovarian cancer.

2. The method of claim 1, wherein the ovarian cancer is an epithelial ovarian cancer.

3. The method of claim 1, wherein the ovarian cancer is a stromal ovarian cancer.

4. The method of claim 1, wherein the ovarian cancer is a germ cell ovarian cancer.

5. The method of claim 1, wherein occurrence of the MMPFF peptide in the urine is assessed by contacting the urine with a first antibody that binds specifically with the MMPFF peptide and assessing whether the MMPFF peptide has bound with the first antibody.

6. The method of claim 5, wherein the urine is centrifuged to substantially remove any sediment therefrom prior to contacting the urine with the first antibody.

7. The method of claim 5, wherein the first antibody is bound to a substrate.

8. The method of claim 7, wherein the substrate is a plastic container.

9. The method of claim 8, wherein the container is a multi-well plate.

10. The method of claim 9, wherein the plate is adapted for automated analysis by a robotic apparatus.

11. The method of claim 5, wherein binding of the MMPFF peptide and the first antibody is assessed by contacting the first antibody with a second antibody that binds specifically with the MMPFF peptide after contacting the first antibody with the urine and assessing co-localization of the first and second antibodies.

12. The method of claim 11, wherein the urine is contacted with the first antibody for at least 10 minutes before contacting the first antibody with the second antibody.

13. The method of claim 11, wherein the urine is contacted with the first antibody for at least 60 minutes before contacting the first antibody with the second antibody.

14. The method of claim 11, wherein the second antibody is detectably labeled.

15. The method of claim 14, wherein the second antibody is linked with a compound selected from the group consisting of an enzyme, a radionuclide, a fluorophore, and a chromophore.

16. The method of claim 11, wherein the second antibody is linked to a ligand and wherein co-localization of the first and second antibodies is assessed by contacting the second antibody with a compound comprising (i) a receptor that binds with the ligand and (ii) a detectable label.

17. The method of claim 16, wherein the label is selected from the group consisting of an enzyme, a radionuclide, a fluorophore, and a chromophore.

18. The method of claim 16, wherein the ligand is biotin and the receptor is an avidin.

19. The method of claim 18, wherein the avidin is streptavidin.

20. The method of claim 19, wherein the label is an enzyme.

21. The method of claim 20, wherein the enzyme is horseradish peroxidase.

22. The method of claim 21, wherein co-localization of the first and second antibodies is assessed in the presence of 3,3',5,5'-tetramethylbenzidine.

23. The method of claim 5, wherein the first antibody is contacted with a labeled substrate of the first antibody after contacting the first antibody with the urine and comparing the amount of labeled ligand that binds with the first antibody that has been contacted with the urine and the amount of labeled ligand that binds with an equivalent amount of the first antibody that has not been contacted with the urine.

24. The method of claim 23, wherein the labeled ligand has the same amino acid sequence as the MMPFF peptide.

25. The method of claim 24, wherein the glycosylation state of the labeled ligand is the same as the glycosylation state of the MMPFF peptide.

26. The method of claim 1, wherein the MMPFF peptide is glycosylated.

27. The method of claim 1, wherein the amino acid sequence of the MMPFF peptide comprises at least 10 consecutive residues of a sequence selected from the group consisting; of SEQ ID NOs: 1-5.

28. The method of claim 27, wherein the amino acid sequence of the MMPFF peptide comprises at least 20 consecutive residues of a sequence selected from the group consisting of SEQ ID NOs: 1-5.

29. The method of claim 27, wherein the amino acid sequence of the MMPFF peptide comprises at least 50 consecutive residues of a sequence selected from the group consisting of SEQ ID NOs: 1-5.

30. The method of claim 27, wherein the amino acid sequence of the MMPFF peptide comprises at least 200 consecutive residues of a sequence selected from the group consisting of SEQ ID NOs: 1-5.

31. The method of claim 27, wherein the amino acid sequence of the MMPFF peptide comprises a sequence selected from the group consisting of SEQ ID NOs: 1-5.

32. The method of claim 27, wherein the amino acid sequence of the MMPFF peptide comprises at least 10 consecutive residues of SEQ ID NO: 4.

33. The method of claim 27, wherein the amino acid sequence of the MMPFF peptide comprises at least 10 consecutive residues of SEQ ID NO: 5.

34. The method of claim 1, further comprising assessing occurrence of a second ovarian cancer marker in the urine of the woman, whereby occurrence of the second marker in the urine is also an indication that the woman is afflicted with an ovarian cancer.

35. The method of claim 34, wherein the second ovarian cancer marker is selected from the group consisting of urinary gonadotropin peptide, cysteine proteinase, neopterin, and tumor-associated trypsin inhibitor.

36. The method of claim 1, further comprising assessing occurrence of a second ovarian cancer marker in the serum of the woman, whereby occurrence of the second marker in the serum is also an indication that the woman is afflicted with an ovarian cancer.

37. The method of claim 36, wherein the second ovarian cancer marker is selected from the group consisting of CA 125, carcinoembryonic antigen, vascular endothelial growth factors tumor-associated trypsin inhibitor, a CD44 splice variant, anti-malignin antibody, lactate dehydrogenase, alpha-hydroxybutyrate dehydrogenase, CA19-9, tissue polypeptide antigen, and sialyl TN.

38. The method of claim 36, wherein the second ovarian cancer marker is CA 125.

39. The method of claim 1, further comprising assessing occurrence of the MMPFF peptide in the serum of the woman, whereby occurrence of the MMPFF peptide in the serum is also an indication that the woman is afflicted with an ovarian cancer.

40. A method of assessing the likelihood that a woman will develop an ovarian cancer, the method comprising assessing occurrence of a mesothelin/megakaryocyte potentiating factor family (MMPFF) peptide in urine obtained from the woman, whereby occurrence of the MMPFF peptide in the urine is an indication that the woman is more likely to develop an ovarian cancer than an otherwise identical woman in whose urine the MMPFF does not occur.

41. A method of assessing the efficacy of a therapy for an ovarian cancer in a woman, the method comprising assessing the amount of a mesothelin/megakaryocyte potentiating factor family (MMPFF) peptide in urine obtained from the woman following the therapy and comparing that amount with the amount of the MMPFF in urine obtained from the woman before the therapy, whereby a lower amount of the MMPFF peptide in the urine following the therapy is an indication that the therapy is efficacious.

## Patentansprüche

1. Verfahren zum Diagnostizieren von Eierstock-Krebs in einer Frau, das Verfahren umfassend das Beurteilen des Auftretens eines MMPFF (mesothelin/megakaryocyte potentiating factor family)-Peptids im Urin, welcher von der Frau erhalten worden ist, wobei das Auftreten des MMPFF-Peptids im Urin eine Indikation dafür ist, dass die Frau mit einem Eierstock-Krebs behaftet ist.

2. Verfahren nach Anspruch 1, wobei der Eierstock-Krebs ein epithelialer Eierstock-Krebs ist.

3. Verfahren nach Anspruch 1, wobei der Eierstock-Krebs ein Stroma-Eierstock-Krebs ist.

4. Verfahren nach Anspruch 1, wobei der Eierstock-Krebs ein Keimzell-Eierstock-Krebs ist.

5. Verfahren nach Anspruch 1, wobei das Auftreten des MMPFF-Peptids in dem Urin beurteilt wird durch Inkontaktbringen des Urins mit einem ersten Antikörper, welcher spezifisch mit dem MMPFF-Peptid bindet, und Bewerten, ob das MMPFF-Peptid mit dem ersten Antikörper gebunden hat.

6. Verfahren nach Anspruch 5, wobei der Urin zentrifugiert ist, um jegliches Sediment daraus vor dem Inkontaktbringen des Urins mit dem ersten Antikörper im Wesentlichen zu entfernen.

7. Verfahren nach Anspruch 5, wobei der erste Antikörper an ein Substrat gebunden ist.

8. Verfahren nach Anspruch 7, wobei das Substrat ein Plastikbehälter ist.

9. Verfahren nach Anspruch 8, wobei der Behälter eine Multiwell-Platte ist.

10. Verfahren nach Anspruch 9, wobei die Platte für die automatisierte Analyse mittels eines Roboters geeignet ist.

11. Verfahren nach Anspruch 5, wobei das Binden des MMPFF-Peptids und des ersten Antikörpers beurteilt wird durch Inkontaktbringen des ersten Antikörpers mit einem zweiten Antikörper, welcher spezifisch mit dem MMPFF-Peptid nach Inkontaktbringen des ersten Antikörpers mit dem Urin bindet, und Beurteilen der Co-Lokalisation der ersten und zweiten Antikörper.

12. Verfahren nach Anspruch 11, wobei der Urin mit dem ersten Antikörper für mindestens 10 Minuten in Kontakt gebracht wird, bevor der erste Antikörper mit dem zweiten Antikörper in Kontakt gebracht wird.

13. Verfahren nach Anspruch 11, wobei der Urin mit dem ersten Antikörper für mindestens 60 Minuten in Kontakt gebracht wird, bevor der erste Antikörper mit dem zweiten Antikörper in Kontakt gebracht wird.

14. Verfahren nach Anspruch 11, wobei der zweite Antikörper nachweisbar markiert ist.

15. Verfahren nach Anspruch 14, wobei der zweite Antikörper mit einer Verbindung verbunden ist, welche ausgewählt ist aus der Gruppe bestehend aus einem Enzym, einem Radionuklid, einem Fluorophor und einem Chromophor.

16. Verfahren nach Anspruch 11, wobei der zweite Antikörper mit einem Liganden verbunden ist, und wobei die Co-Lokalisation der ersten und zweiten Antikörper beurteilt wird durch Inkontaktbringen des zweiten Antikörpers mit einer Verbindung umfassend (i) einen Rezeptor, welcher mit dem Liganden bindet, und (ii) einer nachweisbaren Markierung.

17. Verfahren nach Anspruch 16, wobei die Markierung ausgewählt ist aus der Gruppe bestehend aus einem Enzym, einem Radionuklid, einem Fluorophor und einem Chromophor.

18. Verfahren nach Anspruch 16, wobei der Ligand Biotin und der Rezeptor Avidin ist.

19. Verfahren nach Anspruch 18, wobei das Avidin Streptavidin ist.

20. Verfahren nach Anspruch 19, wobei die Markierung ein Enzym ist.

21. Verfahren nach Anspruch 20, wobei das Enzym Meerrettich-Peroxidase ist.

22. Verfahren nach Anspruch 21, wobei die Co-Lokalisation der ersten und zweiten Antikörper in der Gegenwart von 3,3',5,5'-Tetramethylbenzidin beurteilt wird.

23. Verfahren nach Anspruch 5, wobei der erste Antikörper in Kontakt gebracht wird mit einem markierten Substrat des ersten Antikörpers nach Inkontaktbringen des ersten Antikörpers mit dem Urin und Vergleichen der Menge an markiertem Ligand, welche mit dem ersten Antikörper bindet, welcher mit dem Urin in Kontakt gebracht worden ist, und der Menge an markiertem Ligand, welche mit einer äquivalenten Menge des ersten Antikörpers bindet, welcher nicht mit dem Urin in Kontakt gebracht worden ist.

24. Verfahren nach Anspruch 23, wobei der markierte Ligand die gleiche Aminosäure-Sequenz wie das MMPFF-Peptid hat.

25. Verfahren nach Anspruch 24, wobei der Glykosylierungs-Zustand des markierten Liganden der gleiche ist wie der Glykosylierungs-Zustand des MMPFF-Peptids.

26. Verfahren nach Anspruch 1, wobei das MMPFF-Peptid glykosyliert ist.

27. Verfahren nach Anspruch 1, wobei die Aminosäuresequenz des MMPFF-Peptids mindestens 10 aufeinander folgende Reste einer Sequenz ausgewählt aus der Gruppe bestehend aus den SEQ ID NRn. 1 bis 5 umfasst.

28. Verfahren nach Anspruch 27, wobei die Aminosäuresequenz des MMPFF-Peptids mindestens 20 aufeinander folgende Reste einer Sequenz ausgewählt aus der Gruppe bestehend aus den SEQ ID NRn. 1 bis 5 umfasst.

29. Verfahren nach Anspruch 27, wobei die Aminosäuresequenz des MMPFF-Peptids mindestens 50 aufeinander folgende Reste einer Sequenz ausgewählt aus der Gruppe bestehend aus den SEQ ID NRn. 1 bis 5 umfasst.

30. Verfahren nach Anspruch 27, wobei die Aminosäuresequenz des MMPFF-Peptids mindestens 200 aufeinander folgende Reste einer Sequenz ausgewählt aus der Gruppe bestehend aus den SEQ ID NRn. 1 bis 5 umfasst.

31. Verfahren nach Anspruch 27, wobei die Aminosäuresequenz des MMPFF-Peptids eine Sequenz ausgewählt aus der Gruppe bestehend aus den SEQ ID NRn. 1 bis 5 umfasst.

32. Verfahren nach Anspruch 27, wobei die Aminosäuresequenz des MMPFF-Peptids mindestens 10 aufeinander folgende Reste von SEQ ID NR. 4 umfasst.

33. Verfahren nach Anspruch 27, wobei die Aminosäuresequenz des MMPFF-Peptids mindestens 10 aufeinander folgende Reste von SEQ ID NR. 5 umfasst.

34. Verfahren nach Anspruch 1, weiterhin umfassend das Beurteilen des Auftretens eines zweiten Markers für Eierstock-Krebs in dem Urin der Frau, wobei das Auftreten des zweiten Markers in dem Urin ebenfalls eine Indikation dafür ist, dass die Frau mit einem Eierstock-Krebs behaftet ist.

35. Verfahren nach Anspruch 34, wobei der zweite Marker für Eierstock-Krebs ausgewählt ist aus der Gruppe bestehend aus Urin-Gonadotropin-Peptid, Cystein-Proteinase, Neopterin und Tumor-assoziiertem Trypsin-Inhibitor.

36. Verfahren nach Anspruch 1, weiterhin umfassend das Beurteilen des Auftretens eines zweiten Markers für Eierstock-Krebs in dem Serum der Frau, wobei das Auftreten des zweiten Markers in dem Serum ebenfalls eine Indikation dafür ist, dass die Frau mit einem Eierstock-Krebs behaftet ist.

37. Verfahren nach Anspruch 36, wobei der zweite Marker für Eierstock-Krebs ausgewählt ist aus der Gruppe bestehend aus CA 125, karzinoembryonalem Antigen, vaskulärendothelialen Wachstumsfaktoren, Tumor-assoziiertem Trypsin-Inhibitor, einer CD44 Splice-Variante, Anti-Malignin-Antikörper, Laktat-Dehydrogenase, Alpha-Hydroxybutyrat-Dehydrogenase, CA19-9, Gewebe-Polypeptid-Antigen und Sialyl-TN.

38. Verfahren nach Anspruch 36, wobei der der zweite Marker für Eierstock-Krebs CA 125 ist.

39. Verfahren nach Anspruch 1, weiterhin umfassend das Beurteilen des Auftretens des MMPFF-Peptids im Serum der Frau, wobei das Auftreten des MMPFF-Peptids in dem Serum der Frau ebenfalls eine Indikation dafür ist, dass die Frau mit einem Eierstock-Krebs behaftet ist.

40. Verfahren zum Bewerten der Wahrscheinlichkeit, dass eine Frau einen Eierstock-Krebs entwickeln wird, das Verfahren umfassend das Beurteilen des Auftretens eines MMPFF (mesothelin/megakaryocyte potentiating factor family)-Peptids im Urin, welcher von der Frau erhalten worden ist, wobei das Auftreten des MMPFF-Peptids im Urin eine Indikation dafür ist, dass die Frau eher einen Eierstock-Krebs entwickelt als eine ansonsten identische Frau, in deren Urin das MMPFF nicht auftritt.

41. Verfahren zum Bewerten der Wirksamkeit einer Therapie für einen Eierstock-Krebs in einer Frau, das Verfahren umfassend das Beurteilen der Menge eines MMPFF (mesothelin/megakaryocyte potentiating factor family)-Peptids im Urin, welcher von einer Frau nach der Therapie erhalten worden ist, und Vergleichen dieser Menge mit der Menge des MMPFF im Urin, welcher von der Frau vor der Therapie erhalten worden ist, wobei eine geringere Menge des MMPFF-Peptids in dem Urin nach der Therapie eine Indikation dafür ist, dass die Therapie wirksam ist.

## Revendications

1. Procédé pour diagnostiquer un cancer ovarien chez une femme, ledit procédé comprenant la détermination de l'apparition d'un peptide de la famille du facteur de potentialisation de mésothéline/mégacaryocytes dans l'urine obtenue à partir de la femme, l'apparition du peptide MMPFF dans l'urine étant une indication que la femme est atteinte d'un cancer ovarien.

2. Procédé selon la revendication 1, dans lequel le cancer ovarien est un cancer ovarien épithélial.

3. Procédé selon la revendication 1, dans lequel le cancer ovarien est un cancer ovarien stromal.

4. Procédé selon la revendication 1, dans lequel le cancer ovarien est un cancer ovarien à cellules germinales.

5. Procédé selon la revendication 1, dans lequel l'apparition du peptide MMPFF dans l'urine est déterminée par mise en contact de l'urine avec un premier anticorps qui se lie spécifiquement au peptide MMPFF et par l'opération consistant à déterminer si le peptide MMPFF s'est lié au premier anticorps.

6. Procédé selon la revendication 5, dans lequel l'urine est centrifugée pour que pratiquement tout sédiment en soit éliminé avant mise en contact de l'urine avec le premier anticorps.

7. Procédé selon la revendication 5, dans lequel le premier anticorps est lié à un substrat.

8. Procédé selon la revendication 7, dans lequel le substrat est un récipient en matière plastique.

9. Procédé selon la revendication 8, dans lequel le récipient est une plaque à puits multiples.

10. Procédé selon la revendication 9, dans lequel la plaque est adaptée pour une analyse automatisée par un dispositif robotisé.

11. Procédé selon la revendication 5, dans lequel la liaison du peptide MMPFF et du premier anticorps est déterminée par mise en contact du premier anticorps avec un second anticorps qui se lie spécifiquement au peptide MMPFF après mise en contact du premier anticorps avec l'urine et par détermination de la co-localisation des premier et second anticorps.

12. Procédé selon la revendication 11, dans lequel l'urine est mise en contact avec le premier anticorps pendant au moins 10 minutes avant mise en contact du premier anticorps avec le second anticorps.

13. Procédé selon la revendication 11, dans lequel l'urine est mise en contact avec le premier anticorps pendant au moins 60 minutes avant mise en contact du premier anticorps avec le second anticorps.

14. Procédé selon la revendication 11, dans lequel le second anticorps est marqué de façon détectable.

15. Procédé selon la revendication 14, dans lequel le second anticorps est lié à un composé choisi dans l'ensemble constitué par une enzyme, un radionucléide, un fluorophore, et un chromophore.

16. Procédé selon la revendication 11, dans lequel le second anticorps est lié à un ligand et dans lequel la co-localisation des premier et second anticorps est déterminée par mise en contact du second anticorps avec un composé comprenant (i) un récepteur qui se lie au ligand et (ii) un marqueur détectable.

17. Procédé selon la revendication 16, dans lequel le marqueur est choisi dans l'ensemble constitué par une enzyme, un radionucléide, un fluorophore, et un chromophore.

18. Procédé selon la revendication 16, dans lequel le ligand est la biotine et le récepteur est une avidine.

19. Procédé selon la revendication 18, dans lequel l'avidine est la streptavidine.

20. Procédé selon la revendication 19, dans lequel le marqueur est une enzyme.

21. Procédé selon la revendication 20, dans lequel l'enzyme est la peroxydase de raifort.

22. Procédé selon la revendication 21, dans lequel la co-localisation des premier et second anticorps est déterminée en présence de 3,3',5,5'-tétraméthylbenzidine.

23. Procédé selon la revendication 5, dans lequel le premier anticorps est mis en contact avec un substrat marqué du premier anticorps après que le premier anticorps ait été mis en contact avec l'urine, et comparaison de la quantité de ligand marqué qui se lie au premier anticorps qui a été mis en contact avec l'urine et de la quantité de ligand marqué qui se lie avec une quantité équivalente du premier anticorps qui n'a pas été mis en contact avec l'urine.

24. Procédé selon la revendication 23, dans lequel le ligand marqué a la même séquence d'acides aminés que le peptide MMPFF.

25. Procédé selon la revendication 24, dans lequel l'état de glycosylation du ligand marqué est identique à l'état de glycosylation du peptide MMPFF.

26. Procédé selon la revendication 1, dans lequel le peptide MMPFF est glycosylé.

27. Procédé selon la revendication 1, dans lequel la séquence d'acides aminés du peptide MMPFF comprend au moins 10 résidus consécutifs d'une séquence choisie dans l'ensemble constitué par les SEQ ID NO : 1 à 5.

28. Procédé selon la revendication 27, dans lequel la séquence d'acides aminés du peptide MMPFF comprend au moins 20 résidus consécutifs d'une séquence choisie dans l'ensemble constitué par les SEQ ID NO : 1 à 5.

29. Procédé selon la revendication 27, dans lequel la séquence d'acides aminés du peptide MMPFF comprend au moins 50 résidus consécutifs d'une séquence choisie dans l'ensemble constitué par les SEQ ID NO : 1 à 5.

30. Procédé selon la revendication 27, dans lequel la séquence d'acides aminés du peptide MMPFF comprend au moins 200 résidus consécutifs d'une séquence choisie dans l'ensemble constitué par les SEQ ID NO : 1 à 5.

31. Procédé selon la revendication 27, dans lequel la séquence d'acides aminés du peptide MMPFF comprend une séquence choisie dans l'ensemble constitué par les SEQ ID NO : 1 à 5.

32. Procédé selon la revendication 27, dans lequel la séquence d'acides aminés du peptide MMPFF comprend au moins 10 résidus consécutifs de la SEQ ID NO : 4.

33. Procédé selon la revendication 27, dans lequel la séquence d'acides aminés du peptide MMPFF comprend au moins 10 résidus consécutifs de la SEQ ID NO : 5.

34. Procédé selon la revendication 1, comprenant en outre la détermination de l'apparition d'un second marqueur de cancer ovarien dans l'urine de la femme, l'apparition du second marqueur dans l'urine étant également une indication que la femme est atteinte d'un cancer ovarien.

35. Procédé selon la revendication 34, dans lequel le deuxième marqueur de cancer ovarien est choisi dans l'ensemble constitué par le peptide de gonadotrophine urinaire, la cystéine protéinase, la néoptérine, et l'inhibiteur de trypsine associé à une tumeur.

36. Procédé selon la revendication 1, comprenant en outre la détermination de l'apparition d'un second marqueur de cancer ovarien dans le sérum de la femme, l'apparition du second marqueur dans le sérum étant également une indication que la femme est atteinte d'un cancer ovarien.

37. Procédé selon la revendication 36, dans lequel le second marqueur de cancer ovarien est choisi dans l'ensemble constitué par CA 125, l'antigène carcino-embryonnaire, les facteurs de croissance endothéliaux vasculaires, l'inhibiteur de trypsine associé à une tumeur, un variant d'épissage de CD44, un anticorps anti-malignine, une lactate déshydrogénase, l'a-hydroxybutyrate déshydrogénase, le CA19-9, un antigène de polypeptide tissulaire, et le sialyl-TN.

38. Procédé selon la revendication 36, dans lequel le second marqueur de cancer ovarien est le CA 125.

39. Procédé selon la revendication 1, comprenant en outre la détermination de l'apparition du peptide MMPFF dans le sérum de la femme, l'apparition du peptide MMPFF dans le sérum étant également une indication que la femme est atteinte d'un cancer ovarien.

40. Procédé pour déterminer la susceptibilité qu'aura une femme de développer un cancer ovarien, ledit procédé comprenant la détermination de l'apparition d'un peptide de la famille du facteur de potentialisation de mésothéline/mégacaryocytes (MMPFF) dans l'urine obtenue à partir de la femme, l'apparition du peptide MMPFF dans l'urine étant une indication que la femme est plus susceptible de développer un cancer ovarien qu'une femme par ailleurs identique dans l'urine de laquelle n'apparaît pas le MMPFF.

41. Procédé pour déterminer l'efficacité d'un traitement d'un cancer ovarien chez une femme, ledit procédé comprenant la détermination de la quantité d'un peptide de la famille du facteur de potentialisation de mésothéline/mégacaryocytes (MMPFF) dans l'urine obtenue à partir de la femme après traitement, et la comparaison de cette quantité avec la quantité de MMPFF dans l'urine obtenue à partir de la femme avant le traitement, une quantité inférieure du peptide MMPFF dans l'urine après le traitement étant une indication de l'efficacité du traitement.


### SEQUENCE LISTING

<110> FUJIREBIO AMERICA, INC.
<120> DETECTION OF URINARY MESOTHELIN-/MEGAKARYOCYTE POTENTIATING FACTOR-RELATED PEPTIDES FOR ASSESSMENT OF OVARIAN CANCER
<130> EP36246HV900peu
<140> EP 04 720 803.8
   <141> 2005-10-13
<150> PCT/US2004/007765
   <151> 2004-03-15
<150> US 10/388,930
   <151> 2003-03-13
<160> 5
<170> PatentIn version 3.2
<210> 1
   <211> 628
   <212> PRT
   <213> Homo sapiens
<400> 1
   Met Ala Leu Gln Arg Leu Asp Pro Cys Trp Ser Cys Gly Asp Arg Pro
   1               5                   10                  15
   Gly Ser Leu Leu Phe Leu Leu Phe Ser Leu Gly Trp Val His Pro Ala
               20                  25                  30
   Arg Thr Leu Ala Gly Glu Thr Gly Thr Glu Ser Ala Pro Leu Gly Gly
           35                  40                  45
   Val Leu Thr Thr Pro His Asn Ile Ser Ser Leu Ser Pro Arg Gln Leu
       50                  55                  60
   Leu Gly Phe Pro Cys Ala Glu Val Ser Gly Leu Ser Thr Glu Arg Val
   65                  70                  75                  80
   Arg Glu Leu Ala Val Ala Leu Ala Gln Lys Asn Val Lys Leu Ser Thr
                   85                  90                  95
   Glu Gln Leu Arg Cys Leu Ala His Arg Leu Ser Glu Pro Pro Glu Asp
               100                 105                 110
   Leu Asp Ala Leu Pro Leu Asp Leu Leu Leu Phe Leu Asn Pro Asp Ala
           115                 120                 125
   Phe Ser Gly Pro Gln Ala Cys Thr Arg Phe Phe Ser Arg Ile Thr Lys
       130                 135                 140
   Ala Asn Val Asp Leu Leu Pro Arg Gly Ala Pro Glu Arg Gln Arg Leu
   145                 150                 155                 160
   Leu Pro Ala Ala Leu Ala Cys Trp Gly Val Arg Gly Ser Leu Leu Ser
                   165                 170                 175
   Glu Ala Asp Val Arg Ala Leu Gly Gly Leu Ala Cys Asp Leu Pro Gly
               180                 185                 190
   Arg Phe Val Ala Glu Ser Ala Glu Val Leu Leu Pro Arg Leu Val Ser
           195                 200                 205
   Cys Pro Gly Pro Leu Asp Gln Asp Gln Gln Glu Ala Ala Arg Ala Ala
       210                 215                 220
   Leu Gln Gly Gly Gly Pro Pro Tyr Gly Pro Pro Ser Thr Trp Ser Val
   225                 230                 235                 240
   Ser Thr Met Asp Ala Leu Arg Gly Leu Leu Pro Val Leu Gly Gln Pro
                   245                 250                 255
   Ile Ile Arg Ser Ile Pro Gln Gly Ile Val Ala Ala Trp Arg Gln Arg
               260                 265                 270
   Ser Ser Arg Asp Pro Ser Trp Arg Gln Pro Glu Arg Thr Ile Leu Arg
           275                 280                 285
   Pro Arg Phe Arg Arg Glu Val Glu Lys Thr Ala Cys Pro Ser Gly Lys
       290                 295                 300
   Lys Ala Arg Glu Ile Asp Glu Ser Leu Ile Phe Tyr Lys Lys Trp Glu
   305                 310                 315                 320
   Leu Glu Ala Cys Val Asp Ala Ala Leu Leu Ala Thr Gln Met Asp Arg
                   325                 330                 335
   Val Asn Ala Ile Pro Phe Thr Tyr Glu Gln Leu Asp Val Leu Lys His
               340                 345                 350
   Lys Leu Asp Glu Leu Tyr Pro Gln Gly Tyr Pro Glu Ser Val Ile Gln
           355                 360                 365
   His Leu Gly Tyr Leu Phe Leu Lys Met Ser Pro Glu Asp Ile Arg Lys
       370                 375                 380
   Trp Asn Val Thr Ser Leu Glu Thr Leu Lys Ala Leu Leu Glu Val Asp
   385                 390                 395                 400
   Lys Gly His Glu Met Ser Pro Gln Ala Pro Arg Arg Pro Leu Pro Gln
                   405                 410                 415
   Val Ala Thr Leu Ile Asp Arg Phe Val Lys Gly Arg Gly Gln Leu Asp
               420                 425                 430
   Lys Asp Thr Leu Asp Thr Leu Thr Ala Phe Tyr Pro Gly Tyr Leu Cys
   435 440 445
   Ser Leu Ser Pro Glu Glu Leu Ser Ser Val Pro Pro Ser Ser Ile Trp
   450 455 460
   Ala Val Arg Pro Gln Asp Leu Asp Thr Cys Asp Pro Arg Gln Leu Asp
   465 470 475 480
   Val Leu Tyr Pro Lys Ala Arg Leu Ala Phe Gln Asn Met Asn Gly Ser
   485 490 495
   Glu Tyr Phe Val Lys Ile Gln Ser Phe Leu Gly Gly Ala Pro Thr Glu
   500 505 510
   Asp Leu Lys Ala Leu Ser Gln Gln Asn Val Ser Met Asp Leu Ala Thr
   515 520 525
   Phe Met Lys Leu Arg Thr Asp Ala Val Leu Pro Leu Thr Val Ala Glu
   530 535 540
   Val Gln Lys Leu Leu Gly Pro His Val Glu Gly Leu Lys Ala Glu Glu
   545 550 555 560
   Arg His Arg Pro Val Arg Asp Trp Ile Leu Arg Gln Arg Gln Asp Asp
   565 570 575
   Leu Asp Thr Leu Gly Leu Gly Leu Gln Gly Gly Ile Pro Asn Gly Tyr
   580 585 590
   Leu Val Leu Asp Leu Ser Val Gln Glu Thr Leu Ser Gly Thr Pro Cys
   595 600 605
   Leu Leu Gly Pro Gly Pro Val Leu Thr Val Leu Ala Leu Leu Leu Ala
   610 615 620
   Ser Thr Leu Ala
   625
<210> 2
   <211> 622
   <212> PRT
   <213> Homo sapiens
<400> 2
   Met Ala Leu Pro Thr Ala Arg Pro Leu Leu Gly Ser Cys Gly Thr Pro
   1               5                   10                  15
   Ala Leu Gly Ser Leu Leu Phe Leu Leu Phe Ser Leu Gly Trp Val Gln
               20                  25                  30
   Pro Ser Arg Thr Leu Ala Gly Glu Thr Gly Gln Glu Ala Ala Pro Leu
           35                  40                  45
   Asp Gly Val Leu Ala Asn Pro Pro Asn Ile Ser Ser Leu Ser Pro Arg
       50                  55                  60
   Gln Leu Leu Gly Phe Pro Cys Ala Glu Val Ser Gly Leu Ser Thr Glu
   65                  70                  75                  80
   Arg Val Arg Glu Leu Ala Val Ala Leu Ala Gln Lys Asn Val Lys Leu
                   85                  90                  95
   Ser Thr Glu Gln Leu Arg Cys Leu Ala His Arg Leu Ser Glu Pro Pro
               100                 105                 110
   Glu Asp Leu Asp Ala Leu Pro Leu Asp Leu Leu Leu Phe Leu Asn Pro
           115                 120                 125
   Asp Ala Phe Ser Gly Pro Gln Ala Cys Thr Arg Phe Phe Ser Arg Ile
       130                 135                 140
   Thr Lys Ala Asn Val Asp Leu Leu Pro Arg Gly Ala Pro Glu Arg Gln
   145                 150                 155                 160
   Arg Leu Leu Pro Ala Ala Leu Ala Cys Trp Gly Val Arg Gly Ser Leu
                   165                 170                 175
   Leu Ser Glu Ala Asp Val Arg Ala Leu Gly Gly Leu Ala Cys Asp Leu
               180                 185                 190
   Pro Gly Arg Phe Val Ala Glu Ser Ala Glu Val Leu Leu Pro Arg Leu
           195                 200                 205
   Val Ser Cys Pro Gly Pro Leu Asp Gln Asp Gln Gln Glu Ala Ala Arg
       210                 215                 220
   Ala Ala Leu Gln Gly Gly Gly Pro Pro Tyr Gly Pro Pro Ser Thr Trp
   225                 230                 235                 240
   Ser Val Ser Thr Met Asp Ala Leu Arg Gly Leu Leu Pro Val Leu Gly
                   245                 250                 255
   Gln Pro Ile Ile Arg Ser Ile Pro Gln Gly Ile Val Ala Ala Trp Arg
               260                 265                 270
   Gln Arg Ser Ser Arg Asp Pro Ser Trp Arg Gln Pro Glu Arg Thr Ile
           275                 280                 285
   Leu Arg Pro Arg Phe Arg Arg Glu Val Glu Lys Thr Ala Cys Pro Ser
       290                 295                 300
   Gly Lys Lys Ala Arg Glu Ile Asp Glu Ser Leu Ile Phe Tyr Lys Lys
   305                 310                 315                 320
   Trp Glu Leu Glu Ala Cys Val Asp Ala Ala Leu Leu Ala Thr Gln Met
                   325                 330                 335
   Asp Arg Val Asn Ala Ile Pro Phe Thr Tyr Glu Gln Leu Asp Val Leu
               340                 345                 350
   Lys His Lys Leu Asp Glu Leu Tyr Pro Gln Gly Tyr Pro Glu Ser Val
           355                 360                 365
   Ile Gln His Leu Gly Tyr Leu Phe Leu Lys Met Ser Pro Glu Asp Ile
       370                 375                 380
   Arg Lys Trp Asn Val Thr Ser Leu Glu Thr Leu Lys Ala Leu Leu Glu
   385                 390                 395                 400
   Val Asn Lys Gly His Glu Met Ser Pro Gln Val Ala Thr Leu Ile Asp
                   405                 410                 415
   Arg Phe Val Lys Gly Arg Gly Gln Leu Asp Lys Asp Thr Leu Asp Thr
               420                 425                 430
   Leu Thr Ala Phe Tyr Pro Gly Tyr Leu Cys Ser Leu Ser Pro Glu Glu
           435                 440                 445
   Leu Ser Ser Val Pro Pro Ser Ser Ile Trp Ala Val Arg Pro Gln Asp
       450                 455                 460
   Leu Asp Thr Cys Asp Pro Arg Gln Leu Asp Val Leu Tyr Pro Lys Ala
   465                 470                 475                 480
   Arg Leu Ala Phe Gln Asn Met Asn Gly Ser Glu Tyr Phe Val Lys Ile
                   485                 490                 495
   Gln Ser Phe Leu Gly Gly Ala Pro Thr Glu Asp Leu Lys Ala Leu Ser
               500                 505                 510
   Gln Gln Asn Val Ser Met Asp Leu Ala Thr Phe Met Lys Leu Arg Thr
           515                 520                 525
   Asp Ala Val Leu Pro Leu Thr Val Ala Glu Val Gln Lys Leu Leu Gly
       530                 535                 540
   Pro His Val Glu Gly Leu Lys Ala Glu Glu Arg His Arg Pro Val Arg
   545                 550                 555                 560
   Asp Trp Ile Leu Arg Gln Arg Gln Asp Asp Leu Asp Thr Leu Gly Leu
                   565                 570                 575
   Gly Leu Gln Gly Gly Ile Pro Asn Gly Tyr Leu Val Leu Asp Leu Ser
               580                 585                 590
   Val Gln Glu Ala Leu Ser Gly Thr Pro Cys Leu Leu Gly Pro Gly Pro
   595 600 605
   Val Leu Thr Val Leu Ala Leu Leu Leu Ala Ser Thr Leu Ala
   610 615 620
<210> 3
   <211> 398
   <212> PRT
   <213> Homo sapiens
<400> 3
   Val Glu Lys Thr Ala Cys Pro Ser Gly Lys Lys Ala Arg Glu Ile Asp
   1               5                   10                  15
   Glu Ser Leu Ile Phe Tyr Lys Lys Trp Glu Leu Glu Ala Cys Val Asp
               20                  25                  30
   Ala Ala Leu Leu Ala Thr Gln Met Asp Arg Val Asn Ala Ile Pro Phe
           35                  40                  45
   Thr Tyr Glu Gln Leu Asp Val Leu Lys His Lys Leu Asp Glu Leu Tyr
       50                  55                  60
   Pro Gln Gly Tyr Pro Glu Ser Val Ile Gln His Leu Gly Tyr Leu Phe
   65                  70                  75                  80
   Leu Lys Met Ser Pro Glu Asp Ile Arg Lys Trp Asn Val Thr Ser Leu
                   85                  90                  95
   Glu Thr Leu Lys Ala Leu Leu Glu Val Asn Lys Gly His Glu Met Ser
               100                 105                 110
   Pro Gln Val Ala Thr Leu Ile Asp Arg Phe Val Lys Gly Arg Gly Gln
           115                 120                 125
   Leu Asp Lys Asp Thr Leu Asp Thr Leu Thr Ala Phe Tyr Pro Gly Tyr
       130                 135                 140
   Leu Cys Ser Leu Ser Pro Glu Glu Leu Ser Ser Val Pro Pro Ser Ser
   145                 150                 155                 160
   Ile Trp Ala Val Arg Pro Gln Asp Leu Asp Thr Cys Asp Pro Arg Gln
                   165                 170                 175
   Leu Asp Val Leu Tyr Pro Lys Ala Arg Leu Ala Phe Gln Asn Met Asn
               180                 185                 190
   Gly Ser Glu Tyr Phe Val Lys Ile Gln Ser Phe Leu Gly Gly Ala Pro
           195                 200                 205
   Thr Glu Asp Leu Lys Ala Leu Ser Gln Gln Asn Val Ser Met Asp Leu
       210                 215                 220
   Ala Thr Phe Met Lys Leu Arg Thr Asp Ala Val Leu Pro Leu Thr Val
   225 230 235 240
   Ala Glu Val Gln Lys Leu Leu Gly Pro His Val Glu Gly Leu Lys Ala
   245 250 255
   Glu Glu Arg His Arg Pro Val Arg Asp Trp Ile Leu Arg Gln Arg Gln
   260 265 270
   Asp Asp Leu Asp Thr Leu Gly Leu Gly Leu Gln Gly Gly Ile Pro Asn
   275 280 285
   Gly Tyr Leu Val Leu Asp Leu Ser Val Gln Gly Gly Arg Gly Gly Gln
   290 295 300
   Ala Arg Ala Gly Gly Arg Ala Gly Gly Val Glu Val Gly Ala Leu Ser
   305 310 315 320
   His Pro Ser Leu Cys Arg Gly Pro Leu Gly Asp Ala Leu Pro Pro Arg
   325 330 335
   Thr Trp Thr Cys Ser His Arg Pro Gly Thr Ala Pro Ser Leu His Pro
   340 345 350
   Gly Leu Arg Ala Pro Leu Pro Cys Trp Pro Gln Pro Cys Trp Gly Ser
   355 360 365
   Pro Pro Gly Gln Glu Gln Ala Arg Val Ile Pro Val Pro Pro Gln Glu
   370 375 380
   Asn Ser Arg Ser Val Asn Gly Asn Met Pro Pro Ala Asp Thr
   385 390 395
<210> 4
   <211> 307
   <212> PRT
   <213> artificial sequence
<220>
   <223> Preferred epitopes for generation of antibodies
<220>
   <221> VARIANT
   <222> (107)..(107)
   <223> xaa is any amino acid residue
<400> 4
   Glu Val Glu Lys Thr Ala Cys Pro Ser Gly Lys Lys Ala Arg Glu Ile
   1 5 10 15
   Asp Glu Ser Leu Ile Phe Tyr Lys Lys Trp Glu Leu Glu Ala Cys Val
   20 25 30
   Asp Ala Ala Leu Leu Ala Thr Gln Met Asp Arg Val Asn Ala Ile Pro
           35                  40                  45
   Phe Thr Tyr Glu Gln Leu Asp Val Leu Lys His Lys Leu Asp Glu Leu
       50                  55                  60
   Tyr Pro Gln Gly Tyr Pro Glu Ser Val Ile Gln His Leu Gly Tyr Leu
   65                  70                  75                  80
   Phe Leu Lys Met Ser Pro Glu Asp Ile Arg Lys Trp Asn Val Thr Ser
                   85                  90                  95
   Leu Glu Thr Leu Lys Ala Leu Leu Glu Val Xaa Lys Gly His Glu Met
               100                 105                 110
   Ser Pro Gln Ala Pro Arg Arg Pro Leu Pro Gln Val Ala Thr Leu Ile
           115                 120                 125
   Asp Arg Phe Val Lys Gly Arg Gly Gln Leu Asp Lys Asp Thr Leu Asp
       130                 135                 140
   Thr Leu Thr Ala Phe Tyr Pro Gly Tyr Leu Cys Ser Leu Ser Pro Glu
   145                 150                 155                 160
   Glu Leu Ser Ser Val Pro Pro Ser Ser Ile Trp Ala Val Arg Pro Gln
                   165                 170                 175
   Asp Leu Asp Thr Cys Asp Pro Arg Gln Leu Asp Val Leu Tyr Pro Lys
               180                 185                 190
   Ala Arg Leu Ala Phe Gln Asn Met Asn Gly Ser Glu Tyr Phe Val Lys
           195                 200                 205
   Ile Gln Ser Phe Leu Gly Gly Ala Pro Thr Glu Asp Leu Lys Ala Leu
       210                 215                 220
   Ser Gln Gln Asn Val Ser Met Asp Leu Ala Thr Phe Met Lys Leu Arg
   225                 230                 235                 240
   Thr Asp Ala Val Leu Pro Leu Thr Val Ala Glu Val Gln Lys Leu Leu
                   245                 250                 255
   Gly Pro His Val Glu Gly Leu Lys Ala Glu Glu Arg His Arg Pro Val
               260                 265                 270
   Arg Asp Trp Ile Leu Arg Gln Arg Gln Asp Asp Leu Asp Thr Leu Gly
           275                 280                 285
   Leu Gly Leu Gln Gly Gly Ile Pro Asn Gly Tyr Leu Val Leu Asp Leu
       290                 295                 300
   Ser Val Gln
<210> 5
   <211> 299
   <212> PRT
   <213> artificial
<220>
   <223> Preferred epitopes for generation of antibodies
<220>
   <221> VARIANT
   <222> (107)..(107)
   <223> Xaa is any amino acid residue
<400> 5
   Glu Val Glu Lys Thr Ala Cys Pro Ser Gly Lys Lys Ala Arg Glu Ile
   1               5                   10                  15
   Asp Glu Ser Leu Ile Phe Tyr Lys Lys Trp Glu Leu Glu Ala Cys Val
               20                  25                  30
   Asp Ala Ala Leu Leu Ala Thr Gln Met Asp Arg Val Asn Ala Ile Pro
           35                  40                  45
   Phe Thr Tyr Glu Gln Leu Asp Val Leu Lys His Lys Leu Asp Glu Leu
       50                  55                  60
   Tyr Pro Gln Gly Tyr Pro Glu Ser Val Ile Gln His Leu Gly Tyr Leu
   65                  70                  75                  80
   Phe Leu Lys Met Ser Pro Glu Asp Ile Arg Lys Trp Asn Val Thr Ser
                   85                  90                  95
   Leu Glu Thr Leu Lys Ala Leu Leu Glu Val Xaa Lys Gly His Glu Met
               100                 105                 110
   Ser Pro Gln Val Ala Thr Leu Ile Asp Arg Phe Val Lys Gly Arg Gly
           115                 120                 125
   Gln Leu Asp Lys Asp Thr Leu Asp Thr Leu Thr Ala Phe Tyr Pro Gly
       130                 135                 140
   Tyr Leu Cys Ser Leu Ser Pro Glu Glu Leu Ser Ser Val Pro Pro Ser
   145                 150                 155                 160
   Ser Ile Trp Ala Val Arg Pro Gln Asp Leu Asp Thr Cys Asp Pro Arg
                   165                 170                 175
   Gln Leu Asp Val Leu Tyr Pro Lys Ala Arg Leu Ala Phe Gln Asn Met
               180                 185                 190
   Asn Gly Ser Glu Tyr Phe Val Lys Ile Gln Ser Phe Leu Gly Gly Ala
           195                 200                 205
   Pro Thr Glu Asp Leu Lys Ala Leu Ser Gln Gln Asn Val Ser Met Asp
       210                 215                 220
   Leu Ala Thr Phe Met Lys Leu Arg Thr Asp Ala Val Leu Pro Leu Thr
   225                 230                 235                 240
   Val Ala Glu Val Gln Lys Leu Leu Gly Pro His Val Glu Gly Leu Lys
                   245                 250                 255
   Ala Glu Glu Arg His Arg Pro Val Arg Asp Trp Ile Leu Arg Gln Arg
               260                 265                 270
   Gln Asp Asp Leu Asp Thr Leu Gly Leu Gly Leu Gln Gly Gly Ile Pro
           275                 280                 285
   Asn Gly Tyr Leu Val Leu Asp Leu Ser Val Gln
       290                 295

### SEQUENCE LISTING

<110> FUJIREBIO AMERICA, INC.
<120> DETECTION OF URINARY MESOTHELIN-/MEGAKARYOCYTE POTENTIATING FACTOR-RELATED PEPTIDES FOR ASSESSMENT OF OVARIAN CANCER
<130> EP36246HV900peu
<140> EP 04 720 803.8
   <141> 2005-10-13
<150> PCT/US2004/007765
   <151> 2004-03-15
<150> US 10/388,930
   <151> 2003-03-13
<160> 5
<170> PatentIn version 3.2
<210> 1
   <211> 628
   <212> PRT
   <213> Homo sapiens
<400> 1
   Met Ala Leu Gln Arg Leu Asp Pro Cys Trp Ser Cys Gly Asp Arg Pro
   1               5                   10                  15
   Gly Ser Leu Leu Phe Leu Leu Phe Ser Leu Gly Trp Val His Pro Ala
               20                  25                  30
   Arg Thr Leu Ala Gly Glu Thr Gly Thr Glu Ser Ala Pro Leu Gly Gly
           35                  40                  45
   Val Leu Thr Thr Pro His Asn Ile Ser Ser Leu Ser Pro Arg Gln Leu
       50                  55                  60
   Leu Gly Phe Pro Cys Ala Glu Val Ser Gly Leu Ser Thr Glu Arg Val
   65                  70                  75                  80
   Arg Glu Leu Ala Val Ala Leu Ala Gln Lys Asn Val Lys Leu Ser Thr
                   85                  90                  95
   Glu Gln Leu Arg Cys Leu Ala His Arg Leu Ser Glu Pro Pro Glu Asp
               100                 105                 110
   Leu Asp Ala Leu Pro Leu Asp Leu Leu Leu Phe Leu Asn Pro Asp Ala
           115                 120                 125
   Phe Ser Gly Pro Gln Ala Cys Thr Arg Phe Phe Ser Arg Ile Thr Lys
       130                 135                 140
   Ala Asn Val Asp Leu Leu Pro Arg Gly Ala Pro Glu Arg Gln Arg Leu
   145                 150                 155                 160
   Leu Pro Ala Ala Leu Ala Cys Trp Gly Val Arg Gly Ser Leu Leu Ser
   165 170 175
   Glu Ala Asp Val Arg Ala Leu Gly Gly Leu Ala Cys Asp Leu Pro Gly
   180 185 190
   Arg Phe Val Ala Glu Ser Ala Glu Val Leu Leu Pro Arg Leu Val Ser
   195 200 205
   Cys Pro Gly Pro Leu Asp Gln Asp Gln Gln Glu Ala Ala Arg Ala Ala
   210 215 220
   Leu Gln Gly Gly Gly Pro Pro Tyr Gly Pro Pro Ser Thr Trp Ser Val
   225 230 235 240
   Ser Thr Met Asp Ala Leu Arg Gly Leu Leu Pro Val Leu Gly Gln Pro
   245 250 255
   Ile Ile Arg Ser Ile Pro Gln Gly Ile Val Ala Ala Trp Arg Gln Arg
   260 265 270
   Ser Ser Arg Asp Pro Ser Trp Arg Gln Pro Glu Arg Thr Ile Leu Arg
   275 280 285
   Pro Arg Phe Arg Arg Glu Val Glu Lys Thr Ala Cys Pro Ser Gly Lys
   290 295 300
   Lys Ala Arg Glu Ile Asp Glu Ser Leu Ile Phe Tyr Lys Lys Trp Glu
   305 310 315 320
   Leu Glu Ala Cys Val Asp Ala Ala Leu Leu Ala Thr Gln Met Asp Arg
   325 330 335
   Val Asn Ala Ile Pro Phe Thr Tyr Glu Gln Leu Asp Val Leu Lys His
   340 345 350
   Lys Leu Asp Glu Leu Tyr Pro Gln Gly Tyr Pro Glu Ser Val Ile Gln
   355 360 365
   His Leu Gly Tyr Leu Phe Leu Lys Met Ser Pro Glu Asp Ile Arg Lys
   370 375 380
   Trp Asn Val Thr Ser Leu Glu Thr Leu Lys Ala Leu Leu Glu Val Asp
   385 390 395 400
   Lys Gly His Glu Met Ser Pro Gln Ala Pro Arg Arg Pro Leu Pro Gln
   405 410 415
   Val Ala Thr Leu Ile Asp Arg Phe Val Lys Gly Arg Gly Gln Leu Asp
   420 425 430
   Lys Asp Thr Leu Asp Thr Leu Thr Ala Phe Tyr Pro Gly Tyr Leu Cys
               435                 440                 445
   Ser Leu Ser Pro Glu Glu Leu Ser Ser Val Pro Pro Ser Ser Ile Trp
       450                 455                 460
   Ala Val Arg Pro Gln Asp Leu Asp Thr Cys Asp Pro Arg Gln Leu Asp
   465                 470                 475                 480
   Val Leu Tyr Pro Lys Ala Arg Leu Ala Phe Gln Asn Met Asn Gly Ser
                   485                 490                 495
   Glu Tyr Phe Val Lys Ile Gln Ser Phe Leu Gly Gly Ala Pro Thr Glu
               500                 505                 510
   Asp Leu Lys Ala Leu Ser Gln Gln Asn Val Ser Met Asp Leu Ala Thr
           515                 520                 525
   Phe Met Lys Leu Arg Thr Asp Ala Val Leu Pro Leu Thr Val Ala Glu
       530                 535                 540
   Val Gln Lys Leu Leu Gly Pro His Val Glu Gly Leu Lys Ala Glu Glu
   545                 550                 555                 560
   Arg His Arg Pro Val Arg Asp Trp Ile Leu Arg Gln Arg Gln Asp Asp
                   565                 570                 575
   Leu Asp Thr Leu Gly Leu Gly Leu Gln Gly Gly Ile Pro Asn Gly Tyr
               580                 585                 590
   Leu Val Leu Asp Leu Ser Val Gln Glu Thr Leu Ser Gly Thr Pro Cys
           595                 600                 605
   Leu Leu Gly Pro Gly Pro Val Leu Thr Val Leu Ala Leu Leu Leu Ala
       610                 615                 620
   Ser Thr Leu Ala
   625
<210> 2
   <211> 622
   <212> PRT
   <213> Homo sapiens
<400> 2
   Met Ala Leu Pro Thr Ala Arg Pro Leu Leu Gly Ser Cys Gly Thr Pro
   1               5                   10                  15
   Ala Leu Gly Ser Leu Leu Phe Leu Leu Phe Ser Leu Gly Trp Val Gln
               20                  25                  30
   Pro Ser Arg Thr Leu Ala Gly Glu Thr Gly Gln Glu Ala Ala Pro Leu
           35                  40                  45
   Asp Gly Val Leu Ala Asn Pro Pro Asn Ile Ser Ser Leu Ser Pro Arg
       50                  55                  60
   Gln Leu Leu Gly Phe Pro Cys Ala Glu Val Ser Gly Leu Ser Thr Glu
   65                  70                  75                  80
   Arg Val Arg Glu Leu Ala Val Ala Leu Ala Gln Lys Asn Val Lys Leu
                   85                  90                  95
   Ser Thr Glu Gln Leu Arg Cys Leu Ala His Arg Leu Ser Glu Pro Pro
               100                 105                 110
   Glu Asp Leu Asp Ala Leu Pro Leu Asp Leu Leu Leu Phe Leu Asn Pro
           115                 120                 125
   Asp Ala Phe Ser Gly Pro Gln Ala Cys Thr Arg Phe Phe Ser Arg Ile
       130                 135                 140
   Thr Lys Ala Asn Val Asp Leu Leu Pro Arg Gly Ala Pro Glu Arg Gln
   145                 150                 155                 160
   Arg Leu Leu Pro Ala Ala Leu Ala Cys Trp Gly Val Arg Gly Ser Leu
                   165                 170                 175
   Leu Ser Glu Ala Asp Val Arg Ala Leu Gly Gly Leu Ala Cys Asp Leu
               180                 185                 190
   Pro Gly Arg Phe Val Ala Glu Ser Ala Glu Val Leu Leu Pro Arg Leu
           195                 200                 205
   Val Ser Cys Pro Gly Pro Leu Asp Gln Asp Gln Gln Glu Ala Ala Arg
       210                 215                 220
   Ala Ala Leu Gln Gly Gly Gly Pro Pro Tyr Gly Pro Pro Ser Thr Trp
   225                 230                 235                 240
   Ser Val Ser Thr Met Asp Ala Leu Arg Gly Leu Leu Pro Val Leu Gly
                   245                 250                 255
   Gln Pro Ile Ile Arg Ser Ile Pro Gln Gly Ile Val Ala Ala Trp Arg
               260                 265                 270
   Gln Arg Ser Ser Arg Asp Pro Ser Trp Arg Gln Pro Glu Arg Thr Ile
           275                 280                 285
   Leu Arg Pro Arg Phe Arg Arg Glu Val Glu Lys Thr Ala Cys Pro Ser
       290                 295                 300
   Gly Lys Lys Ala Arg Glu Ile Asp Glu Ser Leu Ile Phe Tyr Lys Lys
   305                 310                 315                 320
   Trp Glu Leu Glu Ala Cys Val Asp Ala Ala Leu Leu Ala Thr Gln Met
   325 330 335
   Asp Arg Val Asn Ala Ile Pro Phe Thr Tyr Glu Gln Leu Asp Val Leu
   340 345 350
   Lys His Lys Leu Asp Glu Leu Tyr Pro Gln Gly Tyr Pro Glu Ser Val
   355 360 365
   Ile Gln His Leu Gly Tyr Leu Phe Leu Lys Met Ser Pro Glu Asp Ile
   370 375 380
   Arg Lys Trp Asn Val Thr Ser Leu Glu Thr Leu Lys Ala Leu Leu Glu
   385 390 395 400
   Val Asn Lys Gly His Glu Met Ser Pro Gln Val Ala Thr Leu Ile Asp
   405 410 415
   Arg Phe Val Lys Gly Arg Gly Gln Leu Asp Lys Asp Thr Leu Asp Thr
   420 425 430
   Leu Thr Ala Phe Tyr Pro Gly Tyr Leu Cys Ser Leu Ser Pro Glu Glu
   435 440 445
   Leu Ser Ser Val Pro Pro Ser Ser Ile Trp Ala Val Arg Pro Gln Asp
   450 455 460
   Leu Asp Thr Cys Asp Pro Arg Gln Leu Asp Val Leu Tyr Pro Lys Ala
   465 470 475 480
   Arg Leu Ala Phe Gln Asn Met Asn Gly Ser Glu Tyr Phe Val Lys Ile
   485 490 495
   Gln Ser Phe Leu Gly Gly Ala Pro Thr Glu Asp Leu Lys Ala Leu Ser
   500 505 510
   Gln Gln Asn Val Ser Met Asp Leu Ala Thr Phe Met Lys Leu Arg Thr
   515 520 525
   Asp Ala Val Leu Pro Leu Thr Val Ala Glu Val Gln Lys Leu Leu Gly
   530 535 540
   Pro His Val Glu Gly Leu Lys Ala Glu Glu Arg His Arg Pro Val Arg
   545 550 555 560
   Asp Trp Ile Leu Arg Gln Arg Gln Asp Asp Leu Asp Thr Leu Gly Leu
   565 570 575
   Gly Leu Gln Gly Gly Ile Pro Asn Gly Tyr Leu Val Leu Asp Leu Ser
   580 585 590
   Val Gln Glu Ala Leu Ser Gly Thr Pro Cys Leu Leu Gly Pro Gly Pro
   595 600 605
   Val Leu Thr Val Leu Ala Leu Leu Leu Ala Ser Thr Leu Ala
   610 615 620
<210> 3
   <211> 398
   <212> PRT
   <213> Homo sapiens
<400> 3
   Val Glu Lys Thr Ala Cys Pro Ser Gly Lys Lys Ala Arg Glu Ile Asp
   1               5                   10                  15
   Glu Ser Leu Ile Phe Tyr Lys Lys Trp Glu Leu Glu Ala Cys Val Asp
               20                  25                  30
   Ala Ala Leu Leu Ala Thr Gln Met Asp Arg Val Asn Ala Ile Pro Phe
           35                  40                  45
   Thr Tyr Glu Gln Leu Asp Val Leu Lys His Lys Leu Asp Glu Leu Tyr
       50                  55                  60
   Pro Gln Gly Tyr Pro Glu Ser Val Ile Gln His Leu Gly Tyr Leu Phe
   65                  70                  75                  80
   Leu Lys Met Ser Pro Glu Asp Ile Arg Lys Trp Asn Val Thr Ser Leu
                   85                  90                  95
   Glu Thr Leu Lys Ala Leu Leu Glu Val Asn Lys Gly His Glu Met Ser
               100                 105                 110
   Pro Gln Val Ala Thr Leu Ile Asp Arg Phe Val Lys Gly Arg Gly Gln
           115                 120                 125
   Leu Asp Lys Asp Thr Leu Asp Thr Leu Thr Ala Phe Tyr Pro Gly Tyr
       130                 135                 140
   Leu Cys Ser Leu Ser Pro Glu Glu Leu Ser Ser Val Pro Pro Ser Ser
   145                 150                 155                 160
   Ile Trp Ala Val Arg Pro Gln Asp Leu Asp Thr Cys Asp Pro Arg Gln
                   165                 170                 175
   Leu Asp Val Leu Tyr Pro Lys Ala Arg Leu Ala Phe Gln Asn Met Asn
               180                 185                 190
   Gly Ser Glu Tyr Phe Val Lys Ile Gln Ser Phe Leu Gly Gly Ala Pro
           195                 200                 205
   Thr Glu Asp Leu Lys Ala Leu Ser Gln Gln Asn Val Ser Met Asp Leu
       210                 215                 220
   Ala Thr Phe Met Lys Leu Arg Thr Asp Ala Val Leu Pro Leu Thr Val
   225 230 235 240
   Ala Glu Val Gln Lys Leu Leu Gly Pro His Val Glu Gly Leu Lys Ala
   245 250 255
   Glu Glu Arg His Arg Pro Val Arg Asp Trp Ile Leu Arg Gln Arg Gln
   260 265 270
   Asp Asp Leu Asp Thr Leu Gly Leu Gly Leu Gln Gly Gly Ile Pro Asn
   275 280 285
   Gly Tyr Leu Val Leu Asp Leu Ser Val Gln Gly Gly Arg Gly Gly Gln
   290 295 300
   Ala Arg Ala Gly Gly Arg Ala Gly Gly Val Glu Val Gly Ala Leu Ser
   305 310 315 320
   His Pro Ser Leu Cys Arg Gly Pro Leu Gly Asp Ala Leu Pro Pro Arg
   325 330 335
   Thr Trp Thr Cys Ser His Arg Pro Gly Thr Ala Pro Ser Leu His Pro
   340 345 350
   Gly Leu Arg Ala Pro Leu Pro Cys Trp Pro Gln Pro Cys Trp Gly Ser
   355 360 365
   Pro Pro Gly Gln Glu Gln Ala Arg Val Ile Pro Val Pro Pro Gln Glu
   370 375 380
   Asn Ser Arg Ser Val Asn Gly Asn Met Pro Pro Ala Asp Thr
   385 390 395
<210> 4
   <211> 307
   <212> PRT
   <213> artificial sequence
<220>
   <223> Preferred epitopes for generation of antibodies
<220>
   <221> VARIANT
   <222> (107)..(107)
   <223> Xaa is any amino acid residue
<400> 4
   Glu Val Glu Lys Thr Ala Cys Pro Ser Gly Lys Lys Ala Arg Glu Ile
   1 5 10 15
   Asp Glu Ser Leu Ile Phe Tyr Lys Lys Trp Glu Leu Glu Ala Cys Val
   20 25 30
   Asp Ala Ala Leu Leu Ala Thr Gln Met Asp Arg Val Asn Ala Ile Pro
           35                  40                  45
   Phe Thr Tyr Glu Gln Leu Asp Val Leu Lys His Lys Leu Asp Glu Leu
       50                  55                  60
   Tyr Pro Gln Gly Tyr Pro Glu Ser Val Ile Gln His Leu Gly Tyr Leu
   65                  70                  75                  80
   Phe Leu Lys Met Ser Pro Glu Asp Ile Arg Lys Trp Asn Val Thr Ser
                   85                  90                  95
   Leu Glu Thr Leu Lys Ala Leu Leu Glu Val Xaa Lys Gly His Glu Met
               100                 105                 110
   Ser Pro Gln Ala Pro Arg Arg Pro Leu Pro Gln Val Ala Thr Leu Ile
           115                 120                 125
   Asp Arg Phe Val Lys Gly Arg Gly Gln Leu Asp Lys Asp Thr Leu Asp
       130                 135                 140
   Thr Leu Thr Ala Phe Tyr Pro Gly Tyr Leu Cys Ser Leu Ser Pro Glu
   145                 150                 155                 160
   Glu Leu Ser Ser Val Pro Pro Ser Ser Ile Trp Ala Val Arg Pro Gln
                   165                 170                 175
   Asp Leu Asp Thr Cys Asp Pro Arg Gln Leu Asp Val Leu Tyr Pro Lys
               180                 185                 190
   Ala Arg Leu Ala Phe Gln Asn Met Asn Gly Ser Glu Tyr Phe Val Lys
           195                 200                 205
   Ile Gln Ser Phe Leu Gly Gly Ala Pro Thr Glu Asp Leu Lys Ala Leu
       210                 215                 220
   Ser Gln Gln Asn Val Ser Met Asp Leu Ala Thr Phe Met Lys Leu Arg
   225                 230                 235                 240
   Thr Asp Ala Val Leu Pro Leu Thr Val Ala Glu Val Gln Lys Leu Leu
                   245                 250                 255
   Gly Pro His Val Glu Gly Leu Lys Ala Glu Glu Arg His Arg Pro Val
               260                 265                 270
   Arg Asp Trp Ile Leu Arg Gln Arg Gln Asp Asp Leu Asp Thr Leu Gly
           275                 280                 285
   Leu Gly Leu Gln Gly Gly Ile Pro Asn Gly Tyr Leu Val Leu Asp Leu
       290                 295                 300
   Ser Val Gln
   305
<210> 5
   <211> 299
   <212> PRT
   <213> artificial
<220>
   <223> Preferred epitopes for generation of antibodies
<220>
   <221> VARIANT
   <222> (107)..(107)
   <223> Xaa is any amino acid residue
<400> 5
   Glu Val Glu Lys Thr Ala Cys Pro Ser Gly Lys Lys Ala Arg Glu Ile
   1               5                   10                  15
   Asp Glu Ser Leu Ile Phe Tyr Lys Lys Trp Glu Leu Glu Ala Cys Val
               20                  25                  30
   Asp Ala Ala Leu Leu Ala Thr Gln Met Asp Arg Val Asn Ala Ile Pro
           35                  40                  45
   Phe Thr Tyr Glu Gln Leu Asp Val Leu Lys His Lys Leu Asp Glu Leu
       50                  55                  60
   Tyr Pro Gln Gly Tyr Pro Glu Ser Val Ile Gln His Leu Gly Tyr Leu
   65                  70                  75                  80
   Phe Leu Lys Met Ser Pro Glu Asp Ile Arg Lys Trp Asn Val Thr Ser
                   85                  90                  95
   Leu Glu Thr Leu Lys Ala Leu Leu Glu Val Xaa Lys Gly His Glu Met
               100                 105                 110
   Ser Pro Gln Val Ala Thr Leu Ile Asp Arg Phe Val Lys Gly Arg Gly
           115                 120                 125
   Gln Leu Asp Lys Asp Thr Leu Asp Thr Leu Thr Ala Phe Tyr Pro Gly
       130                 135                 140
   Tyr Leu Cys Ser Leu Ser Pro Glu Glu Leu Ser Ser Val Pro Pro Ser
   145                 150                 155                 160
   Ser Ile Trp Ala Val Arg Pro Gln Asp Leu Asp Thr Cys Asp Pro Arg
                   165                 170                 175
   Gln Leu Asp Val Leu Tyr Pro Lys Ala Arg Leu Ala Phe Gln Asn Met
               180                 185                 190
   Asn Gly Ser Glu Tyr Phe Val Lys Ile Gln Ser Phe Leu Gly Gly Ala
           195                 200                 205
   Pro Thr Glu Asp Leu Lys Ala Leu Ser Gln Gln Asn Val Ser Met Asp
       210                 215                 220
   Leu Ala Thr Phe Met Lys Leu Arg Thr Asp Ala Val Leu Pro Leu Thr
   225                 230                 235                 240
   Val Ala Glu Val Gln Lys Leu Leu Gly Pro His Val Glu Gly Leu Lys
                   245                 250                 255
   Ala Glu Glu Arg His Arg Pro Val Arg Asp Trp Ile Leu Arg Gln Arg
               260                 265                 270
   Gln Asp Asp Leu Asp Thr Leu Gly Leu Gly Leu Gln Gly Gly Ile Pro
           275                 280                 285
   Asn Gly Tyr Leu Val Leu Asp Leu Ser Val Gln
       290                 295

### SEQUENCE LISTING

<110> FUJIREBIO AMERICA, INC.
<120> DETECTION OF URINARY MESOTHELIN-/MEGAKARYOCYTE POTENTIATING FACTOR-RELATED PEPTIDES FOR ASSESSMENT OF OVARIAN CANCER
<130> EP36246HV900peu
<140> EP 04 720 803.8
   <141> 2005-10-13
<150> PCT/US2004/007765
   <151> 2004-03-15
<150> US 10/388,930
   <151> 2003-03-13
<160> 5
<170> PatentIn version 3.2
<210> 1
   <211> 628
   <212> PRT
   <213> Homo sapiens
<400> 1
   Met Ala Leu Gln Arg Leu Asp Pro Cys Trp Ser Cys Gly Asp Arg Pro
   1               5                   10                  15
   Gly Ser Leu Leu Phe Leu Leu Phe Ser Leu Gly Trp Val His Pro Ala
               20                  25                  30
   Arg Thr Leu Ala Gly Glu Thr Gly Thr Glu Ser Ala Pro Leu Gly Gly
           35                  40                  45
   Val Leu Thr Thr Pro His Asn Ile Ser Ser Leu Ser Pro Arg Gln Leu
       50                  55                  60
   Leu Gly Phe Pro Cys Ala Glu Val Ser Gly Leu Ser Thr Glu Arg Val
   65                  70                  75                  80
   Arg Glu Leu Ala Val Ala Leu Ala Gln Lys Asn Val Lys Leu Ser Thr
                   85                  90                  95
   Glu Gln Leu Arg Cys Leu Ala His Arg Leu Ser Glu Pro Pro Glu Asp
               100                 105                 110
   Leu Asp Ala Leu Pro Leu Asp Leu Leu Leu Phe Leu Asn Pro Asp Ala
           115                 120                 125
   Phe Ser Gly Pro Gln Ala Cys Thr Arg Phe Phe Ser Arg Ile Thr Lys
       130                 135                 140
   Ala Asn Val Asp Leu Leu Pro Arg Gly Ala Pro Glu Arg Gln Arg Leu
   145                 150                 155                 160
   Leu Pro Ala Ala Leu Ala Cys Trp Gly Val Arg Gly Ser Leu Leu Ser
   165 170 175
   Glu Ala Asp Val Arg Ala Leu Gly Gly Leu Ala Cys Asp Leu Pro Gly
   180 185 190
   Arg Phe Val Ala Glu Ser Ala Glu Val Leu Leu Pro Arg Leu Val Ser
   195 200 205
   Cys Pro Gly Pro Leu Asp Gln Asp Gln Gln Glu Ala Ala Arg Ala Ala
   210 215 220
   Leu Gln Gly Gly Gly Pro Pro Tyr Gly Pro Pro Ser Thr Trp Ser Val
   225 230 235 240
   Ser Thr Met Asp Ala Leu Arg Gly Leu Leu Pro Val Leu Gly Gln Pro
   245 250 255
   Ile Ile Arg Ser Ile Pro Gln Gly Ile Val Ala Ala Trp Arg Gln Arg
   260 265 270
   Ser Ser Arg Asp Pro Ser Trp Arg Gln Pro Glu Arg Thr Ile Leu Arg
   275 280 285
   Pro Arg Phe Arg Arg Glu Val Glu Lys Thr Ala Cys Pro Ser Gly Lys
   290 295 300
   Lys Ala Arg Glu Ile Asp Glu Ser Leu Ile Phe Tyr Lys Lys Trp Glu
   305 310 315 320
   Leu Glu Ala Cys Val Asp Ala Ala Leu Leu Ala Thr Gln Met Asp Arg
   325 330 335
   Val Asn Ala Ile Pro Phe Thr Tyr Glu Gln Leu Asp Val Leu Lys His
   340 345 350
   Lys Leu Asp Glu Leu Tyr Pro Gln Gly Tyr Pro Glu Ser Val Ile Gln
   355 360 365
   His Leu Gly Tyr Leu Phe Leu Lys Met Ser Pro Glu Asp Ile Arg Lys
   370 375 380
   Trp Asn Val Thr Ser Leu Glu Thr Leu Lys Ala Leu Leu Glu Val Asp
   385 390 395 400
   Lys Gly His Glu Met Ser Pro Gln Ala Pro Arg Arg Pro Leu Pro Gln
   405 410 415
   Val Ala Thr Leu Ile Asp Arg Phe Val Lys Gly Arg Gly Gln Leu Asp
   420 425 430
   Lys Asp Thr Leu Asp Thr Leu Thr Ala Phe Tyr Pro Gly Tyr Leu Cys
           435                 440                 445
   Ser Leu Ser Pro Glu Glu Leu Ser Ser Val Pro Pro Ser Ser Ile Trp
       450                 455                 460
   Ala Val Arg Pro Gln Asp Leu Asp Thr Cys Asp Pro Arg Gln Leu Asp
   465                 470                 475                 480
   Val Leu Tyr Pro Lys Ala Arg Leu Ala Phe Gln Asn Met Asn Gly Ser
                   485                 490                 495
   Glu Tyr Phe Val Lys Ile Gln Ser Phe Leu Gly Gly Ala Pro Thr Glu
               500                 505                 510
   Asp Leu Lys Ala Leu Ser Gln Gln Asn Val Ser Met Asp Leu Ala Thr
           515                 520                 525
   Phe Met Lys Leu Arg Thr Asp Ala Val Leu Pro Leu Thr Val Ala Glu
       530                 535                 540
   Val Gln Lys Leu Leu Gly Pro His Val Glu Gly Leu Lys Ala Glu Glu
   545                 550                 555                 560
   Arg His Arg Pro Val Arg Asp Trp Ile Leu Arg Gln Arg Gln Asp Asp
                   565                 570                 575
   Leu Asp Thr Leu Gly Leu Gly Leu Gln Gly Gly Ile Pro Asn Gly Tyr
               580                 585                 590
   Leu Val Leu Asp Leu Ser Val Gln Glu Thr Leu Ser Gly Thr Pro Cys
           595                 600                 605
   Leu Leu Gly Pro Gly Pro Val Leu Thr Val Leu Ala Leu Leu Leu Ala
       610                 615                 620
   Ser Thr Leu Ala
   625
<210> 2
   <211> 622
   <212> PRT
   <213> Homo sapiens
<400> 2
   Met Ala Leu Pro Thr Ala Arg Pro Leu Leu Gly Ser Cys Gly Thr Pro
   1               5                   10                  15
   Ala Leu Gly Ser Leu Leu Phe Leu Leu Phe Ser Leu Gly Trp Val Gln
               20                  25                  30
   Pro Ser Arg Thr Leu Ala Gly Glu Thr Gly Gln Glu Ala Ala Pro Leu
           35                  40                  45
   Asp Gly Val Leu Ala Asn Pro Pro Asn Ile Ser Ser Leu Ser Pro Arg
       50                  55                  60
   Gln Leu Leu Gly Phe Pro Cys Ala Glu Val Ser Gly Leu Ser Thr Glu
   65                  70                  75                  80
   Arg Val Arg Glu Leu Ala Val Ala Leu Ala Gln Lys Asn Val Lys Leu
                   85                  90                  95
   Ser Thr Glu Gln Leu Arg Cys Leu Ala His Arg Leu Ser Glu Pro Pro
               100                 105                 110
   Glu Asp Leu Asp Ala Leu Pro Leu Asp Leu Leu Leu Phe Leu Asn Pro
           115                 120                 125
   Asp Ala Phe Ser Gly Pro Gln Ala Cys Thr Arg Phe Phe Ser Arg Ile
       130                 135                 140
   Thr Lys Ala Asn Val Asp Leu Leu Pro Arg Gly Ala Pro Glu Arg Gln
   145                 150                 155                 160
   Arg Leu Leu Pro Ala Ala Leu Ala Cys Trp Gly Val Arg Gly Ser Leu
                   165                 170                 175
   Leu Ser Glu Ala Asp Val Arg Ala Leu Gly Gly Leu Ala Cys Asp Leu
               180                 185                 190
   Pro Gly Arg Phe Val Ala Glu Ser Ala Glu Val Leu Leu Pro Arg Leu
           195                 200                 205
   Val Ser Cys Pro Gly Pro Leu Asp Gln Asp Gln Gln Glu Ala Ala Arg
       210                 215                 220
   Ala Ala Leu Gln Gly Gly Gly Pro Pro Tyr Gly Pro Pro Ser Thr Trp
   225                 230                 235                 240
   Ser Val Ser Thr Met Asp Ala Leu Arg Gly Leu Leu Pro Val Leu Gly
                   245                 250                 255
   Gln Pro Ile Ile Arg Ser Ile Pro Gln Gly Ile Val Ala Ala Trp Arg
               260                 265                 270
   Gln Arg Ser Ser Arg Asp Pro Ser Trp Arg Gln Pro Glu Arg Thr Ile
           275                 280                 285
   Leu Arg Pro Arg Phe Arg Arg Glu Val Glu Lys Thr Ala Cys Pro Ser
       290                 295                 300
   Gly Lys Lys Ala Arg Glu Ile Asp Glu Ser Leu Ile Phe Tyr Lys Lys
   305                 310                 315                 320
   Trp Glu Leu Glu Ala Cys Val Asp Ala Ala Leu Leu Ala Thr Gln Met
                   325                 330                 335
   Asp Arg Val Asn Ala Ile Pro Phe Thr Tyr Glu Gln Leu Asp Val Leu
               340                 345                 350
   Lys His Lys Leu Asp Glu Leu Tyr Pro Gln Gly Tyr Pro Glu Ser Val
           355                 360                 365
   Ile Gln His Leu Gly Tyr Leu Phe Leu Lys Met Ser Pro Glu Asp Ile
       370                 375                 380
   Arg Lys Trp Asn Val Thr Ser Leu Glu Thr Leu Lys Ala Leu Leu Glu
   385                 390                 395                 400
   Val Asn Lys Gly His Glu Met Ser Pro Gln Val Ala Thr Leu Ile Asp
                   405                 410                 415
   Arg Phe Val Lys Gly Arg Gly Gln Leu Asp Lys Asp Thr Leu Asp Thr
               420                 425                 430
   Leu Thr Ala Phe Tyr Pro Gly Tyr Leu Cys Ser Leu Ser Pro Glu Glu
           435                 440                 445
   Leu Ser Ser Val Pro Pro Ser Ser Ile Trp Ala Val Arg Pro Gln Asp
       450                 455                 460
   Leu Asp Thr Cys Asp Pro Arg Gln Leu Asp Val Leu Tyr Pro Lys Ala
   465                 470                 475                 480
   Arg Leu Ala Phe Gln Asn Met Asn Gly Ser Glu Tyr Phe Val Lys Ile
                   485                 490                 495
   Gln Ser Phe Leu Gly Gly Ala Pro Thr Glu Asp Leu Lys Ala Leu Ser
               500                 505                 510
   Gln Gln Asn Val Ser Met Asp Leu Ala Thr Phe Met Lys Leu Arg Thr
           515                 520                 525
   Asp Ala Val Leu Pro Leu Thr Val Ala Glu Val Gln Lys Leu Leu Gly
       530                 535                 540
   Pro His Val Glu Gly Leu Lys Ala Glu Glu Arg His Arg Pro Val Arg
   545                 550                 555                 560
   Asp Trp Ile Leu Arg Gln Arg Gln Asp Asp Leu Asp Thr Leu Gly Leu
                   565                 570                 575
   Gly Leu Gln Gly Gly Ile Pro Asn Gly Tyr Leu Val Leu Asp Leu Ser
               580                 585                 590
   Val Gln Glu Ala Leu Ser Gly Thr Pro Cys Leu Leu Gly Pro Gly Pro
   595 600 605
   Val Leu Thr Val Leu Ala Leu Leu Leu Ala Ser Thr Leu Ala
   610 615 620
<210> 3
   <211> 398
   <212> PRT
   <213> Homo sapiens
<400> 3
   Val Glu Lys Thr Ala Cys Pro Ser Gly Lys Lys Ala Arg Glu Ile Asp
   1               5                   10                  15
   Glu Ser Leu Ile Phe Tyr Lys Lys Trp Glu Leu Glu Ala Cys Val Asp
               20                  25                  30
   Ala Ala Leu Leu Ala Thr Gln Met Asp Arg Val Asn Ala Ile Pro Phe
           35                  40                  45
   Thr Tyr Glu Gln Leu Asp Val Leu Lys His Lys Leu Asp Glu Leu Tyr
       50                  55                  60
   Pro Gln Gly Tyr Pro Glu Ser Val Ile Gln His Leu Gly Tyr Leu Phe
   65                  70                  75                  80
   Leu Lys Met Ser Pro Glu Asp Ile Arg Lys Trp Asn Val Thr Ser Leu
                   85                  90                  95
   Glu Thr Leu Lys Ala Leu Leu Glu Val Asn Lys Gly His Glu Met Ser
               100                 105                 110
   Pro Gln Val Ala Thr Leu Ile Asp Arg Phe Val Lys Gly Arg Gly Gln
           115                 120                 125
   Leu Asp Lys Asp Thr Leu Asp Thr Leu Thr Ala Phe Tyr Pro Gly Tyr
       130                 135                 140
   Leu Cys Ser Leu Ser Pro Glu Glu Leu Ser Ser Val Pro Pro Ser Ser
   145                 150                 155                 160
   Ile Trp Ala Val Arg Pro Gln Asp Leu Asp Thr Cys Asp Pro Arg Gln
                   165                 170                 175
   Leu Asp Val Leu Tyr Pro Lys Ala Arg Leu Ala Phe Gln Asn Met Asn
               180                 185                 190
   Gly Ser Glu Tyr Phe Val Lys Ile Gln Ser Phe Leu Gly Gly Ala Pro
           195                 200                 205
   Thr Glu Asp Leu Lys Ala Leu Ser Gln Gln Asn Val Ser Met Asp Leu
       210                 215                 220
   Ala Thr Phe Met Lys Leu Arg Thr Asp Ala Val Leu Pro Leu Thr Val
   225 230 235 240
   Ala Glu Val Gln Lys Leu Leu Gly Pro His Val Glu Gly Leu Lys Ala
   245 250 255
   Glu Glu Arg His Arg Pro Val Arg Asp Trp Ile Leu Arg Gln Arg Gln
   260 265 270
   Asp Asp Leu Asp Thr Leu Gly Leu Gly Leu Gln Gly Gly Ile Pro Asn
   275 280 285
   Gly Tyr Leu Val Leu Asp Leu Ser Val Gln Gly Gly Arg Gly Gly Gln
   290 295 300
   Ala Arg Ala Gly Gly Arg Ala Gly Gly Val Glu Val Gly Ala Leu Ser
   305 310 315 320
   His Pro Ser Leu Cys Arg Gly Pro Leu Gly Asp Ala Leu Pro Pro Arg
   325 330 335
   Thr Trp Thr Cys Ser His Arg Pro Gly Thr Ala Pro Ser Leu His Pro
   340 345 350
   Gly Leu Arg Ala Pro Leu Pro Cys Trp Pro Gln Pro Cys Trp Gly Ser
   355 360 365
   Pro Pro Gly Gln Glu Gln Ala Arg Val Ile Pro Val Pro Pro Gln Glu
   370 375 380
   Asn Ser Arg Ser Val Asn Gly Asn Met Pro Pro Ala Asp Thr
   385 390 395
<210> 4
   <211> 307
   <212> PRT
   <213> artificial sequence
<220>
   <223> Preferred epitopes for generation of antibodies
<220>
   <221> VARIANT
   <222> (107)..(107)
   <223> Xaa is any amino acid residue
<400> 4
   Glu Val Glu Lys Thr Ala Cys Pro Ser Gly Lys Lys Ala Arg Glu Ile
   1 5 10 15
   Asp Glu Ser Leu Ile Phe Tyr Lys Lys Trp Glu Leu Glu Ala Cys Val
   20 25 30
   Asp Ala Ala Leu Leu Ala Thr Gln Met Asp Arg Val Asn Ala Ile Pro
           35                  40                  45
   Phe Thr Tyr Glu Gln Leu Asp Val Leu Lys His Lys Leu Asp Glu Leu
       50                  55                  60
   Tyr Pro Gln Gly Tyr Pro Glu Ser Val Ile Gln His Leu Gly Tyr Leu
   65                  70                  75                  80
   Phe Leu Lys Met Ser Pro Glu Asp Ile Arg Lys Trp Asn Val Thr Ser
                   85                  90                  95
   Leu Glu Thr Leu Lys Ala Leu Leu Glu Val Xaa Lys Gly His Glu Met
               100                 105                 110
   Ser Pro Gln Ala Pro Arg Arg Pro Leu Pro Gln Val Ala Thr Leu Ile
           115                 120                 125
   Asp Arg Phe Val Lys Gly Arg Gly Gln Leu Asp Lys Asp Thr Leu Asp
       130                 135                 140
   Thr Leu Thr Ala Phe Tyr Pro Gly Tyr Leu Cys Ser Leu Ser Pro Glu
   145                 150                 155                 160
   Glu Leu Ser Ser Val Pro Pro Ser Ser Ile Trp Ala Val Arg Pro Gln
                   165                 170                 175
   Asp Leu Asp Thr Cys Asp Pro Arg Gln Leu Asp Val Leu Tyr Pro Lys
               180                 185                 190
   Ala Arg Leu Ala Phe Gln Asn Met Asn Gly Ser Glu Tyr Phe Val Lys
           195                 200                 205
   Ile Gln Ser Phe Leu Gly Gly Ala Pro Thr Glu Asp Leu Lys Ala Leu
       210                 215                 220
   Ser Gln Gln Asn Val Ser Met Asp Leu Ala Thr Phe Met Lys Leu Arg
   225                 230                 235                 240
   Thr Asp Ala Val Leu Pro Leu Thr Val Ala Glu Val Gln Lys Leu Leu
                   245                 250                 255
   Gly Pro His Val Glu Gly Leu Lys Ala Glu Glu Arg His Arg Pro Val
               260                 265                 270
   Arg Asp Trp Ile Leu Arg Gln Arg Gln Asp Asp Leu Asp Thr Leu Gly
           275                 280                 285
   Leu Gly Leu Gln Gly Gly Ile Pro Asn Gly Tyr Leu Val Leu Asp Leu
       290                 295                 300
   Ser Val Gln
   305
<210> 5
   <211> 299
   <212> PRT
   <213> artificial
<220>
   <223> Preferred epitopes for generation of antibodies
<220>
   <221> VARIANT
   <222> (107)..(107)
   <223> Xaa is any amino acid residue
<400> 5
   Glu Val Glu Lys Thr Ala Cys Pro Ser Gly Lys Lys Ala Arg Glu Ile
   1               5                   10                  15
   Asp Glu Ser Leu Ile Phe Tyr Lys Lys Trp Glu Leu Glu Ala Cys Val
               20                  25                  30
   Asp Ala Ala Leu Leu Ala Thr Gln Met Asp Arg Val Asn Ala Ile Pro
           35                  40                  45
   Phe Thr Tyr Glu Gln Leu Asp Val Leu Lys His Lys Leu Asp Glu Leu
       50                  55                  60
   Tyr Pro Gln Gly Tyr Pro Glu Ser Val Ile Gln His Leu Gly Tyr Leu
   65                  70                  75                  80
   Phe Leu Lys Met Ser Pro Glu Asp Ile Arg Lys Trp Asn Val Thr Ser
                   85                  90                  95
   Leu Glu Thr Leu Lys Ala Leu Leu Glu Val Xaa Lys Gly His Glu Met
               100                 105                 110
   Ser Pro Gln Val Ala Thr Leu Ile Asp Arg Phe Val Lys Gly Arg Gly
           115                 120                 125
   Gln Leu Asp Lys Asp Thr Leu Asp Thr Leu Thr Ala Phe Tyr Pro Gly
       130                 135                 140
   Tyr Leu Cys Ser Leu Ser Pro Glu Glu Leu Ser Ser Val Pro Pro Ser
   145                 150                 155                 160
   Ser Ile Trp Ala Val Arg Pro Gln Asp Leu Asp Thr Cys Asp Pro Arg
                   165                 170                 175
   Gln Leu Asp Val Leu Tyr Pro Lys Ala Arg Leu Ala Phe Gln Asn Met
               180                 185                 190
   Asn Gly Ser Glu Tyr Phe Val Lys Ile Gln Ser Phe Leu Gly Gly Ala
           195                 200                 205
   Pro Thr Glu Asp Leu Lys Ala Leu Ser Gln Gln Asn Val Ser Met Asp
       210                 215                 220
   Leu Ala Thr Phe Met Lys Leu Arg Thr Asp Ala Val Leu Pro Leu Thr
   225                 230                 235                 240
   Val Ala Glu Val Gln Lys Leu Leu Gly Pro His Val Glu Gly Leu Lys
                   245                 250                 255
   Ala Glu Glu Arg His Arg Pro Val Arg Asp Trp Ile Leu Arg Gln Arg
               260                 265                 270
   Gln Asp Asp Leu Asp Thr Leu Gly Leu Gly Leu Gln Gly Gly Ile Pro
           275                 280                 285
   Asn Gly Tyr Leu Val Leu Asp Leu Ser Val Gln
       290                 295